# EUROPEAN PATENT APPLICATION

(11) **EP 1 683 419 A1**
(43) Date of publication of application: **26.07.2006**
(21) Application number: 03723494.5
(22) Date of filing: 28.04.2003
(51) Int. Cl.: A01N 63/00, C12P 27/00

(54) **GENERAL ACTIVATOR OF METABOLISM IN PLANTS, ANIMALS AND HUMANS, IN ORDER TO INCREASE AGRICULTURAL AND ANIMAL PRODUCTIVITY AND IMPROVE HUMAN HEALTH**

(71) Applicant: Esparza Alanis, Maria Laura, Monterrey, N.L. (MX)
(72) Inventor: Esparza Alanis, Maria Laura, Monterrey, N.L. (MX)
(74) Representative: McCann, Heather Alison
(86) International application number: PCT/MX2003/000039
(87) International publication number: WO 2004/095931

(57) **Abstract**

The invention relates to a general metabolism activator (AGM) product which is obtained using a fermentative method and which was used to perform chemical and biological tests which revealed the information about the product outlined below. In plants, the product is known as general plant metabolism activator (AGMP) and said product: a) acts as an inhibitor (when an ethyl acetate extraction is performed); b) produces no effects in plants if used directly; and c) stimulates growth and productivity when used in the diluted form, optimum dilutions being 1:1000000000-1:10000000000. In animals, the product is known as general animal metabolism activator (AGMA) and it causes an increase in weight, changes in behaviour and an increase in sexual activity. In humans, the product is known as general human metabolism activator (AGMH) and can be used to relieve fatigue and increase sexual activity. The product also has antioxidant properties. As a result, the product has the following benefits: (1) in plants, the product increases productivity both in terms of quality and quantity, without leaving any toxic waste since it is environment friendly; (2) in animals, the product causes an increase in meat production; (3) in humans, the product relieves fatigue and increases sexual activity; and (4) the product can be used to conserve food and drinks which are likely to undergo oxidation.

## Description

### Field of the invention:

A process for obtaining a product for use in agriculture, livestock and humans by means of a fermentation technique manipulating the composition of the culture substrate as well as its environmental conditions, and the inoculum, which may be fungi, bacteria or yeasts, such as ***Gibberella zeae, Gibberella fujikori, Fusarium moniliforme, Fusarium sp., Azetobacter chroococcum, Agrobacterium tumefaciens, Bacillus polymyxa, Torula pulcherima.***

### State of the art:

### Introduction

Gibberellins are an important large family of diterpenic acids derived biologically from tetracyclic hydrocarbon diterpenes (Fraga 1979 and Graebe and Ropers, cited by Kumar and Lonsane, 1989) which are powerful growth hormones in plants. Of those compounds, the gibberellin GA₃, also known as gibberellic acid (Nakanishi *et al* 1983) has received comparatively more attention from the many scientists who have studied them (Jefferys, 1979). Gibberellic acid, which is widely used to promote plant growth and flowering in agriculture and commercial floriculture is a metabolite of the phytopathogenic fungus Gibberella fujikuroi (the asexual form of which is Fusarium moniliforme), from which it was isolated for the first time in 1935 by Yabuta and Sumiki in Japan (Fraga 1979). Gibberellic acid regulates the rate of growth and the final length of the internodes of plants, amongst other physiological functions (Graebe and Ropers, 1978). To obtain it commercially, industrial manufacturers currently use submerged fermentation techniques although even in the most efficient processes the quantity of gibberellic acid obtained is lower than desirable (Vass and Jefferys 1979). Although it was not possible to find more up-to-date information, the latter scientists were of the opinion that it was possible to reduce the production costs. As far as Mexico is concerned, a semi-exhaustive consultation of different sources shows that the country does not produce GA₃ or gibberellins for commercial purposes. There is evidence, however, that some scientific institutes have conducted, or are conducting, studies to produce them. In view of this situation and the high commercial value of such plant growth regulators, it seems important to study how to improve the existing production techniques.

With the objective of exploring the possiblity of achieving acceptable production of gibberellic acid in an experimental environment and using the hypothesis that it is possible to increase the capacity of the fungus to produce the metabolite by manipulating the composition of the culture substrate, the objective of this study is to produce gibberellic acid from *Fusarium moniliforme* in comparatively greater concentrations than the yields referred to in the literature using inexpensive sources of carbohydrates in a standard culture medium.

### LITERATURE REVIEWED

The historical antecedents of the discovery of gibberellic acid go back to an old phytopathological problem in Japan. In the cultivation of rice there is a disease resulting, in both young and adult plants, in excessive elongation of the stems and in scant fruit. Japanese farmers refer to the disease by a number of names such as bakanae, ajoine, somennae, aganae, sasanae, etc. (Kumar and Lonsane,1989). Early studies relating to the cause of the disease which affects a large number of plant species were carried out by Sawad, who was the first to suggest that it was due to infection by a fungus, later identified as Gibberella fujikuroi (Sawada) Wollenwober (Fraga 1979). In 1926 the symptoms shown by the rice plants were correctly attributed, by Kurosawa (cited by Nakanishi 1983), to a "toxin", later isolated and chemically characterised by Yabuta and Sumiki and Yabuta and Hayashi (cited by Cross 1954). Kurosawa was also able to demonstrate that the sterile filtrates of culture media in which the fungus grew caused the bakanae disease when they were inoculated into stems of young plants. The symptoms of the disease could also be induced in rice plants, the seeds of which had been germinated in the sterile extract of the fungus. Such observations established definitively that the disease was caused by a metabolite produced by the fungus in question (Kumar and Lonsane 1989).

### Description of Fusarium moniliforme

The fungus causing the effect described in the preceding paragraph is a microorganism belonging to the class Deuteromycetes, order Melanconiales, sub-class Hiphomycetidae, family Tuberculariaceae, genus *Fusarium* and species *moniliforme.* All the Deuteromycetes produce spores known as conidia which are produced in sporogenes (Alexopoulos 1979).

Both the sexual form *Gibberella spp* and the asexual form *Fusarium spp.* produce gibberellic acid, which promotes plant growth (MacMillan and Takahashi 1968 and Nakanishi *et al.* 1983).

The plant diseases associated with Gibberella or with *Fusarium* include rotting of the stems, ears and roots, and blight in corn; it also causes scab, blight in plants and rot at the root of species having small-sized grains, such as wheat and other cereals. *Fusarium* produces rot in the roots of beans, asparagus and onion; rot at the foot of pumpkin, dry rot in potatoes and basal rot in stems of numerous ornamental species, as well as rot in the seeds and drowning and blight in plants (Agrios 1989). In particular, the corn diseases caused by *Gibberella* are found widely distributed around the world and cause considerable losses (Agrios 1989).

### Gibberellins

Gibberellins have been isolated from fungi and bacteria (Kumar and Lonsane 1989) and are normal constituents of green plants, in which they occur in tiny quantities (Fraga 1979). In plants that are related taxonomically, structurally similar gibberellins have been identified (Nakamuro and Tsuji 1974). Gibberellins induce notable effects such as promoting growth: they accelerate the elongation of underdeveloped plants so that they catch up to normal size, they promote flowering and elongation of roots and the growth of fruits. Such effects are similar in some aspects to those caused by indoleacetic acid, which also promotes plant growth, the production of which is also induced by gibberellins. Auxins (another class of plant growth regulators) and gibberellins act synergistically. It is very probable that gibberellins activate the release of repressed genes involved in the synthesis of plant growth regulators (Rojas and Ramirez 1987) or by promoting the permeability of the cell membranes to enzymes that stimulate different growth processes (Fraga 1979).

Chemical nature and nomenclature: Gibberellins were extracted for the first time from the fungus Gibberella fujikuroi which, as mentioned, is the causal agent of the "bakanae" disease (which literally means "foolish seedlings" in rice in Japan (Hori cited by Nakanishi *et al* 1983)). The active component isolated and characterised chemically by Yabuta *et al* and Yatazawa and Sumiki (cited by Cross 1954) in the period from the end of the 1930's to the start of the 1950's was called gibberellin B (later gibberellin A) for which the formula C₂₂H₂₆O₇ was proposed (Cross 1954). Cross was unsuccessful, however, in reproducing the results of the Japanese study, but his studies in turn led him to discover a new metabolite of the same fungus, which he called gibberellic acid. The latter also proved to be a powerful plant growth promoter causing elongation of the growth shoots of pea and wheat plants (Brian *et al,* cited by Cross 1954). The chemical characterisation of gibberellic acid (gibberellin GA₃ in accordance with the nomenclature system of Takahashi *et al 1955* and 1957, cited by MacMillan and Takahashi 1968) by crystallography yielded a formula of C₁₉H₂₂O₆ (Cross 1954).

The initial nomenclature of gibberellins caused some confusion owing to the fact that the two most important groups of scientists working on the compounds were using different forms of notation. The first group, represented by Takahashi *et al,* identified gibberellins, in 1955 and 1957, using symbols represented by the letters GA followed by a numerical subscript. Those scientists isolated four gibberellins, which they called GA₁, GA₂, GA₃ and GA₄, from a fermentation medium with *Fusarium moniliforme.* This nomenclature was adopted by MacMillan *et al.,* Cross *et al,* Hanson and Galt (cited by MacMillan and Takahashi 1968). The second group, formed of Tamura *et al* and Mitsui *et al* referred to the gibberellins by the name of the plant where they were discovered, for example, bamboo (corresponding to gibberellin GA₁₉), Pharbitis (gibberellin GA₁₆), Canavalina I (GA₂₁), Lupinus I (GA₁₈), Lupinus II (GA₂₃), etc. (MacMillan and Takahashi 1968). The bamboo gibberellin was isolated later from *Phaseolus multiflorus* seeds, which caused no little confusion. For this reason, many scientists currently tend to use the GA nomenclature, since it has the advantage of being able to add new gibberellins just by allocating them a number. It is important to mention that gibberellin compounds are written with the name of the chemical compound from which they come, followed by the corresponding gibberellin, for example 2-0-acetyl GA₃ (MacMillan and Takahashi 1968).

Some of the early studies enabled the development of methods by isolating the active principle of G. fujikuroi and by its chemical and biological characterisation (Ito and Kimura, and Nisikado, cited by Kumar and Lonsane 1989). However, the techniques for crystallizing the metabolite in pure form were at that time very ineffective (Kumar and Lonsane 1989). Later, around the time from 1961-1963, many attempts were made at chemically synthesizing gibberellins and intermediate compounds using various means (Money *et al,* Kos and Loewenthal, Correy *et al,* Corey and Smith, Corey and Munroe, cited by Kumar and Lonsane 1989). The continuing interest in gibberellins has meant that a large number of gibberellins are now known in plants and in microbial culture media (Jefferys 1969). Those assays have also shown a fair number of chemical properties that can be exploited, such as adsorption, ability to pass through semipermeable membranes and the stability of the metabolite in warm water (Kumar and Lonsane (1989). A publication by Fraga (1979) mentions the identification of 57 gibberellins which can be classified within one of two groups: the C₂₀ gibberellins (which contain the 20 carbon atoms characteristic of a diterpene, the 20-C atom of which can be in the form of methyl, alcohol, aldehyde or acid) and the C₁₉ gibberellins (which have lost the 20-C atom and have the typical lactonic group in the C₁₉-C₂₀ position (Fraga 1979).

### Biological and biochemical effects of gibberellins

Studies into the chemical nature, the biochemical synthesis and the effects of gibberellins on plants saw great impetus towards the end of the 1950's and beginning of the 1960's. Thus, the effects of gibberellins were studied intensively in barley and other crops. Some of the results obtained showed not only increases in the production of barley but also in the production of malt; increases in the size of fruits, such as grapes, breaking of dormancy of seeds and a reduction in the damage caused by frosts (Donoho and Walker, Weaver, Witlwer and Bukovac, Galun, Brian *et al,* Griggs and Iwakiri, cited by Kumar and Lonsane 1989). Another typical effect of GA₃ is the induction of the synthesis of amylase in germinating seeds, making possible the transformation of starch into glucose to generate the energy required for the development of the embryo. According to Jacobsen *et al* (cited by Rojas and Ramirez 1987) this induction occurred by activating an inactive precursor of the corresponding messenger RNA. GA₃ is perhaps also the only hormone that interacts with the phytochrome, the receptor which "tells" the plant the hours of daylight that it receives and makes the plants adjust to the photoperiod for flowering. This effect implies that GA₃ has a role in the mobilisation of the nutrient flow. Moreover, those effects have secondary effects, such as acceleration of germination, flowering outside of the normal photoperiod, development of fruits, etc. (Rojas and Ramirez 1987).

### Industrial production

It was not possible to obtain detailed information relating to the industrial production of gibberellins in Mexico. Nonetheless, it was possible to ascertain that a company in the neighbouring state of Coahuila, which produces and formulates various chemical components of products used in agriculture, has studied the process of fermentation in a liquid medium. It was possible to ascertain that the company sells gibberellic acid imported from China (at a cost of US$ 400-600/kg in 1991) combined with an inert support at a concentration of 10% and a current cost of $8.50 for a 10 g tablet. That company uses analytical chemical confirmation, qualitatively and quantitatively, of the gibberellic acid for quality control by thin-layer chromatography with detection of fluorescence compared with a purified commercial standard. Moreover, they verify the biological effectiveness of the technical material using amylase tests (Fernandez and Johnston 1986), growth of the hypocotyl in lettuce plants (Rovalo and Rojas 1982) and of the second internode of bean plants (Mitchell and Livingston 1973). There follows hereinafter information presented in the specialist literature on the processes for the manufacture of GA₃. An exhaustive review of the subject of fermentation and fermentation techniques can be found in Borrow *et al* (1961).

*Fermentation process:* As in any fermentation, the microbial activity changes substrates rich in carbohydrates (substances predominant in vegetable matter) into alcohols and organic acids, such as gibberellins (Stanier, Doudoroff and Adelberg 1970).

Fermentations used to obtain GA₃ are carried out in the presence of all the necessary nutrients and chemical conditions and optimum temperatures until the fungal growth exhausts the limiting component(s) of the medium (condition called "nutrient limitation") or until conditions develop in which there is insufficient availability of a nutrient (called "nutrient restriction") (Kumar and Lonsane 1989). For the optimum production of GA₃ and other gibberellins there must be a condition of limited nitrogen. During the process of growth of the fungus, gibberellin inhibitors are also synthesized, the presence of which should be avoided in formulating the final product (Borrow *et al* 1961).

The latter scientists have contributed a wealth of information on the process of fermentation for the production of GA₃. The following observations and conclusions drawn from different experiments using various culture media and mycelium of a nonspore-forming *Fusarium moniliforme* strain (Jefferys 1979) were taken from the same article by Borrow *et al* 1961).

The lag phase (which is the time taken for the duplication of the inoculum in the minutes following the addition of the inoculum) was noted only in a medium that contained ammonium acetate, or under conditions of high initial concentration of glucose (30 g/100 ml). There was observed the existence of a period of proliferation in the presence of all the nutrients (balanced phase) during which a unit of growth in dry weight was accompanied by a constant assimilation of glucose, nitrogen, phosphorus, magnesium and potassium. The increase in dry weight was exponential whilst the morphology and chemical composition of the hyphae remained constant in terms of fats, carbohydrates, DNA, RNA and polyols. Under those conditions the cells demonstrate linear exponential growth followed later by decelerated growth. The exponential growth finally stops and a stationary phase begins with the exhaustion of the nitrogen in the presence of glucose. This phase is associated with the production of gibberellins. With the continual presence of glucose administered externally, the "maintenance phase" occurs which continues until the external glucose is exhausted and also until the internal fat reserves are exhausted (but not the carbohydrates). If the glucose is exhausted after the nitrogen then the production of gibberellins can continue for a time, depending upon the length of the storage phase and depending upon the fat reserves available when the reserves have reached approximately the level present in the balanced phase of the mycelium, at which point the "terminal phase" begins. This latter phase is characterized by the breaking down and release of components of the mycelium, phosphate and ammonium into the medium (Jefferys 1979).

*Fermentation techniques:* Initial studies carried out into the production of gibberellins used the "liquid surface fermentation" technique (Yabuta *et al,* cited by Kumar and Lonsane 1989 and Curtis and Cross 1954) but the "submerged fermentation" technique has replaced the former technique and is used much more frequently (Stodola *et al,* cited by Kumar and Lonsane 1989).

The main objective in the commercial production of GA₃ has been to manufacture the product at low cost. This has encouraged the development of studies into various fermentation techniques. Amongst these, the best known techniques are "liquid surface fermentation" or LSF) already mentioned, which requires long periods of incubation and results in low production of GA₃ (Hepner, cited by Kumar and Lonsane 1989), "submerged fermentation" or SmF (Kumar and Lonsane 1989), also already mentioned, "continuous submerged fermentation" which is considered to have the same economic characteristics as continuous fermentation (Holmes and Zacharias, Bu'Lock *et al,* cited by Kumar and Lonsane 1989) and solid-state fermentation (Kumar and Lonsane 1989). Studies into the production of GA₃ using that technique began in the 1980's. In a study carried out by a group of scientists, they mention that there only appear to be economic advantages of that technique (Kumar and Lonsane 1989).
Finally, there is also a production technique using "immobilized cells"; no information as to the economic prospects of that technique has been obtained, although it is reported that certain aspects of that technique are economically positive (Kumar and Lonsane 1988a and 1988b; Heinrich and Rhem; Kahlon and Malhotea, cited by Kumar and Lonsane 1989).
*Other fermentation products:* Numerous authors who have studied the biosynthesis of gibberellins also report many compounds related to them. Amongst them there may be mentioned Wierzchowski and Vierzchoska; Kuhr, Cross *et al;* Schrieber *et al;* Cavell and MacMillan; Serebryakov *et al;* and Pietel *et al,* cited by Kumar and Lonsane 1989. In the culture media used in the production of GA₃, products have been detected that result from the degradation of GA₃, which products have some biological activity in plants (as is the case of isogibberic acid) and many other products including growth promoters, organic acids, amino acids and compounds of diverse nature including enzymes and cofactors. All these compounds, with the exception of the enzymes, are produced normally by the metabolism of the microorganism and some of them are involved in the biosynthesis and degradation of gibberellins (Kumar and Lonsane 1989).

In the fermentations used to produce GA₃ starting from *G. fujikuroi* and *F. moniliforme* other growth promoters are also produced. One of these is FUNGIC ACID which is produced in a concentration 20 times less than gibberellins (Sternberg, cited by Kumar and Lonsane 1989 and Jefferys 1979). Also reported is the production of a plant growth inhibitor by *F. moniliforme* (Kurosawa, cited by Kumar and Lonsane 1989). The suppressor is produced at 35°C whilst the stimulator is produced at 20°C. The suppressor crystallises out and its production is limited to a pH of 3 (Kurosawa and Yabuta *et al,* cited by Kumar and Lonsane 1989). The compound having the trivial name "FUSARIC ACID" was identified as picolinic acid and then as 5-n-butylpicolinic acid (Yabuta *et al* and Yabuta and Hayashi, cited by Kumar and Lonsane 1988). The production of an unidentified inhibitor of plant growth by *Fusarium sp.* was also reported by Thakur and Vyas (1983). In the commercial production of gibberellins it is essential that that repressor of plant growth which is produced concomitantly with the gibberellins is eliminated, whether using selective conditions, such as a low pH, or other appropriate culture conditions (Yabuta *et al* and Kurosawa, cited by Jefferys 1970). Those scientists agree that it is very difficult to inhibit its production.

### Description of the invention:

**A) The product:** A composition: to increase production: agricultural, animal and human.
   Composed of:
   (a) A source of nitrogen, such as : ammonium chloride, ammonium sulphate, ammonium phosphate, sodium nitrate; ammonium tartrate, ammonium acetate. In a quantity of 0.5-20 g/litre
   (b) A source of carbohydrates, such as: glucose, lactose, saccharose, mannose, dextrose, lactose, etc. 10-200 g/litre
   (c) A source of minerals, such as KH₂PO₄, MgSO₄, K₂SO₄, KCl, MgCl₂, K₂HPO₄.
   (d) pH of 5.5 (3-8).
   (e) Incubate at a temperature of 30-45°C, with or without stirring, for 5-20 days, which induces the production of a compound which can be extracted with ethyl acetate and which inhibits the growth of plants when used in concentrated form, and which stimulates the growth of plants from lesser to greater growth progressively according to the dilution from 0.1 to 0.00000000000000000000001, with excellent results being obtained at a concentration of 0.0000000000001.
**B) Use:**
   (a) A UNIQUE product for increasing the productivity of the land, increasing the productivity of animals and contributing to human health.
   (b) A "new" product which is extracted with a solvent and which acts as an inhibitor when directly in contact with plants, but when diluted in extremely small quantities (nanodose) is a general stimulator of plants and animals, the use of the "crude" medium also having been tested in similar dilutions, yielding the same result.
   (c) It may be that the product obtained has either inhibitors or suppressors, such as fusaric acid, which is produced in quantities 20 times less than the gibberellins, but which is formed at high temperatures 35°C whilst gibberellic acid and gibberellins are formed at 20°C. The use of this INHIBITOR in a dilute form results in an excellent General Activator of Metabolism in plants and animals.

### Use in different crops:

A) Citrus fruits
   a) Various tests carried out on this crop showed that solution 5 caused:
   b) an increase in flowering, greater fruit set and therefore increased crop productivity, in some cases an increase in the size of the fruit.
   c) Better quality of fruit, better flavour.
   d) Compared with control, in drought, under the same conditions (without irrigation), resistance to drought was observed. Despite the conditions, fruit was harvested but the fruit was rather small, although very sweet, suitable for making juices.
   d) It was observed that when applied to small citrus plants (size and age), it produces flowering and fruit setting, and the fruit ripens. In winter it was observed that the leafless trees were full of flowers. Frost resistance was also observed.
B) Corn
   In this crop greater plant growth and a greater number of corncobs (up to 5 per plant) were observed. Also improves the quality of the produce since it increases the quantity of proteins therein, larger grains.
**C) Potato**
   Produces an increase in productivity and uniformity in the produce, also greater quantity of good quality potatoes.
**D) Fig trees**
   In this crop an increase in productivity was observed throughout the year, with fruits of great flavor, more compact and less perishable. Cuttings were also taken (small portions of plants were transplanted out of season and the cuttings rooted). More trees obtained.
**F) Pumpkin**
   Increase in quantity, quality and resistance to drought conditions.
**G) Tomato**
   Increase in quantity and quality of the crop, it was also observed that under flood conditions the crop survived and it was possible to use the fruit for making puree; the fruit of control plants was not recoverable (total loss of the crop).
**H) Banana**
   Increases the quantity and quality thereof.
**I) Flowers**
   Gives better quality, greater size and quantity, also observed that flowers survive in the winter (seasonal plants) compared with untreated plants.
**J) Prickly pears**
   Increases the quantity of prickly pears.
**K) Pear trees**
   Notably increases the number of flowers and fruit set in such a manner that the fruit grows in clusters, and improves flavour.
**L) Peach trees**
   Increases the number, size and flavour of the fruit.
**M) Grasses**
   Produces a spectacular increase in the crop, strong roots observed, greener and with more grass, which may be used to feed livestock or for decorative purposes.
**N) Onions.**
   Increases the size and quantity and yields better flavour.
**O) Apples**
   Enables apple production in locations not suited to apple production at high temperatures of more than 40°C; the apples produced have an excellent flavour and are produced by young trees. It is important to mention that apples require the stimulus of cold weather or chemical stimulus to flower and produce fruit.

### Use in plants that are suffering environmentally "stressful" conditions

Helps the plant to resist environmentally "stressful" conditions, such as:
a) salinity - feasible that it increases resistance to drought by developing deeper and stronger roots.
b) acidity (due to acid rain caused by pollution)- greater resistance to this because the plants grow stronger; treated plants survived both cold and heat, or excessive drought.
c) flooding: plants treated with this product in the dilutions mentioned showed crop resistance to flooding, compared with control plants (in the control plants the entire harvest was lost - tested on a tomato crop).
d) helps crops which have erroneously been subjected to "stress" from an agricultural product, such as a herbicide or fertilizer.

### Use of an agricultural product which promotes crops in poor soil or conditions

The result in % productivity of the harvest is more obvious in crops subject to "stress" factors or in crops that have not had fertilizer applied.

### The use of a product to obtain "organic crops or produce".

Use of an environmentally compatible agricultural product does not cause damage to flora, fauna or humans; the crop obtained contains no residues detrimental to human or animal health. This would be a product denoted as "ORGANIC PRODUCE OF THE FIELD".

### Use of a product with high stability when prepared for use in the field:

Use of a prepared product for use in the field has a stability or durability of 3 months.

### Use of a product that is placed on a support:

Such as fabric or cotton, being easy to handle.

### Use of a product to obtain desirable responses in animals and humans, such as the following: General Activator of Metabolism in Animals (GAMA) and General Activator of Metabolism in Humans (GAMH):

Also causes desirable or positive effects in animals and humans in low dilutions, being non-toxic whatever the dilution. The product used is solution 4, diluted in liquors of various flavours, in tequila, in ethyl alcohol, in wines, 4 or 5 drops being administered to the tongue per day; it should be mentioned that if all of the product were taken at once there would be no damaging effects.

Studies were carried out firstly in cats, starting with solution 4 and finishing with a dilution of 1:1000 (solution 16) administered orally; it was observed that the cats increased in size and weight, were always very healthy, and a change in behaviour was also observed in that they appeared more affectionate, their sexual drive appeared to increase as did the number of sexual relations observed.

A study was carried out in chickens, 50 individuals. They ate only during the day (not at night); an increase of 30% in weight was observed and they had good flavour. In domestic chickens which had been given the solution from very young it was observed that some grew very tall (long legs) whilst others did not grow so much but had a large body.
Different behaviour was also observed, they looked after each other more, were always together and were sociable towards the people rearing them.
In dogs, aggression decreases and they grow more healthily.

Solutions were tested from the GRP concentrate to solution 4 in mice and no toxic effect was observed. Great sexual activity was observed, resulting in a large number of litters with no anomalies.

On the basis of these observations, it was concluded that the product was not toxic and that it has other properties worth exploiting.

On that basis, I began to use the product on myself, with solution 4, and observed that I had more energy. After some time, my relations wanted to use it, with similar results. In younger relatives they had a better body, as if they had been exercising, which is why they called the product "fortachon" (tough guy).

Various people used the product and all reported that they had more energy and did not tire easily. Also some acquaintances with impotence problems used it, and reported a return to normality. In normal persons, it causes greater sexual activity in both men and women.

### Use of the product as an antioxidant:

In juices and sour drinks, such as lemon, orange and grapefruit water and their respective juices, upon adding the product at a dilution of 1:1000 - 1:10 000 by adding 0.1 ml it was observed that the solutions did not turn sour and they retained a good flavour for several days compared with controls prepared at the same time and under the same conditions. This same product can be used in foods susceptible to this process, such as apples, avocado and banana.
(Tables A and B were carried out with lemonade and orange juice, respectively.)

**Table A. Lemon water with GAMP in different concentrations, tested on different days and compared with a control.**

| Solution tested Different concentrations of GAMP | Flavour of the lemon water on the first day | Flavour of the lemon water on the second day | Flavour of the lemon water on the third day | Flavour of the lemon water on the fourth day | Flavour of the lemon water on the fifth day | Flavour of the lemon water on the sixth day |
|---|---|---|---|---|---|---|
| control | +++ | - | - | - | - | - |
| 1:1000 | +++ | + | + | - | - | - |
| 1:3000 | +++ | ++ | + | - | - | - |
| 1:7000 | +++ | + | + | - | - | - |
| 1:10 000 | +++ | +++ | ++ | + | - | - - |

| | | | | | | |
|---|---|---|---|---|---|---|
| +++= excellent flavour | | | | | | |
| + + = good flavour | | | | | | |
| + = regular flavour | | | | | | |
| - = bad flavour (bitter) | | | | | | |

**Table B. Orange juice with GAMP in different concentrations, tested on different days and compared with a control.**

| Solution tested | Flavour of the orange juice on the first day | Flavour of the orange juice on the second day | Flavour of the orange juice on the third day | Flavour of the orange juice on the fourth day | Flavour of the orange juice on the fifth day | Flavour of the orange juice on the sixth day |
|---|---|---|---|---|---|---|
| control | +++ | ++ | + | - | - | - |
| 1:1000 | +++ | + | + | + | + | - |
| 1:3000 | +++ | ++ | + | + | ++ | - |
| 1:7000 | +++ | + | + | + | + | - |
| 1: 10000 | +++ | +++ | ++ | ++ | ++ | - |

| | | | | | | |
|---|---|---|---|---|---|---|
| + + + = excellent flavour | | | | | | |
| + + = good flavour | | | | | | |
| + = regular flavour - = bad flavour (bitter) | | | | | | |

### C) Dose:

Use the same dose for all crop varieties, animals and humans, the dose being obtained by making dilutions that obtain the best results at 1:1 000 000 000; 1: 10 000 000 000; 1:100 000 000 000; 1: 1000 000 000 000, 1: 10 000 000 000 0000, dissolved in the quantity of water required to irrigate one hectare; in the case of animals the dose is distributed in the drinking water and in humans the product is diluted in different flavoured liquors, such as wine, tequila etc.

### The following describes the methodology used to obtain the General Activator of Metabolism Product in its different modalities, with the corresponding results

### General Activator of Metabolism in Plants (GAMP):

The purpose of this study was to obtain a product to increase productivity in the field (quantitatively and qualitatively), taking as reference a known growth regulator, gibberellic acid (GA₃), which is obtained by a fermentation process and uses the fungus *Fusarium moniliforme* on different nutrient substrates. The methodology used in carrying out this study consisted of the production, extraction and comparative chemical identification using GA₃ as reference and is produced by the fungus using the submerged fermentation-technique. The study also included testing *in vivo* of the effect of the gibberellic acid obtained on corn seeds and lettuce plants.

In the fermentation process to produce GAMP there were both qualitative and quantitative variations, such as the source of carbohydrates and the fermentation time. Extraction was carried out using ethyl acetate and a solid was obtained which was called GAMPa. A chemical comparison of the two products GAMPa and GA₃ was carried out using thin-layer chromatography (Cross 1954).

This technique showed the presence of a product which is found at the same level in GA₃ but in a different shape (oval for GAMPa) in the different treatments, whereas GA₃ is round. The GAMPa extract obtained with ethyl acetate was subjected to a quantitative assay to determine an *approximate quantity equivalence* between GA₃ and the GAMPa. This is the technique used by Holbrook (1961), which is a spectrophotometric assay and is applied to the GAMPa extracts using chemically pure GA₃ as reference, demonstrating that the GAMPa has a greater equivalence than GA₃. It was found that the greatest quantity of GAMPa was obtained in the treatment 20L15 (20 g/litre of lactose, 15 days' fermentation) with 1125 mg/litre, followed by the treatment 20G7 (20 g/litre of glucose, 7 days' fermentation) with 750 mg/litre. According to the study carried out by high-resolution liquid chromatography, and the quantification by the spectrophotometric technique of Holbrook, there were substantial differences between the different treatments (nature and unit quantity of the substrates used as sources of carbohydrate and incubation time). However, the results of the bioassays into the inducing effect of the GAMPa using GA₃ as positive control on the elongation of the hypocotyl of lettuce plants showed that the average effect of the dilutions tested from GAMPa extracts were more powerful than GA₃. There is evidence that the response of the stems of the lettuce plants, within the range of the dilutions used, depended upon the concentration thereof, although the tendency of the response is not clear because there were more dilutions with positive results at dilutions as low as 1: 10 000 000 000.
Also tested was the effect of the GAMPa solution when prepared in water, and it was found to have a storage durability of 3 months. This advantage is not present in GA₃ since, when a solution of GA₃ is prepared in water, the following day the effect is notably reduced or lost entirely.

In the relative control using standard GA₃, the elongation of the hypocotyl apparently follows an inverse relationship to the increase in the concentration thereof. As to the alpha-amylase activity, in a similar manner there was a different effect amongst the dilutions in the three treatments tested. There also appeared to be an inversely proportional relationship between the growth and the concentration of the phytohormone. This tendency which is inverse to the response was clearly demonstrated in the same test on the stimulation of germination and elongation of lettuce seeds, which was carried out to confirm the tests into the induction of alpha-amylase activity and the same elongation of the above-mentioned hypocotyl.

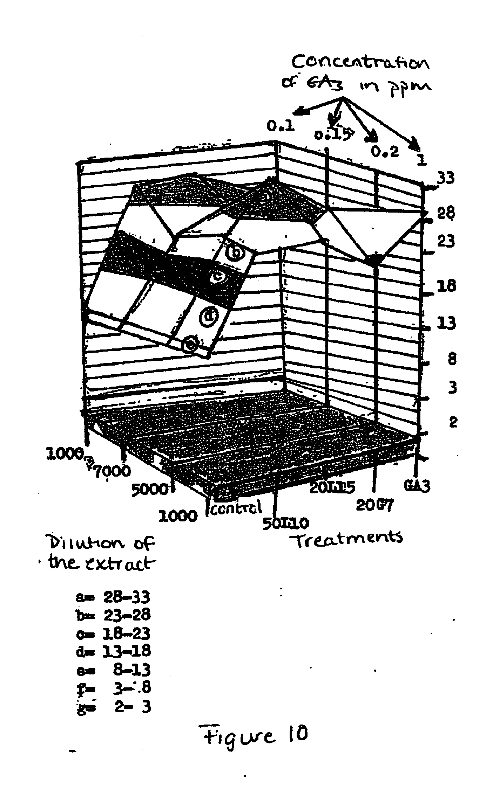

The bioassays of the GAMP, the direct product of the fermentation, showed a positive response in the dilute product, but there was no inhibitory effect in concentrated dilutions, as shown in Figure 11, starting at a dilution of 1:1 000 000 and showing its optimum effect at the lowest dilutions, as shown by solutions 4,5,19,20,21,22,23.

### MATERIALS AND METHODS

A) Methodology to obtain the products GAMP and GAMPa:
   Hereinafter there will be described the methodology, materials and equipment used to carry out the study. Essentially this consisted of the production, extraction and chemical identification and quantification of a Growth Regulating Product GAMP compared with GA₃ gibberellic acid standards produced by a ***Fusarium moniliforme*** strain using the submerged fermentation technique. The study also included testing *in vivo* of the effect of the GAMP obtained on corn seeds and lettuce plants.

### Handling of Fusarium moniliforme

A strain of *Fusarium moniliforme* donated locally was used as source in the production of the General Activator of Plant Growth (GAPG). Mycelium of the fungus sown in PDA was incubated at 30 °C for a period of 48 hours, after which it was kept refrigerated at 2°C. When required, the strain was activated in a standard PDA culture medium, being incubated at 30 °C for 1-3 days in test tubes of 18x150 mm. To extract the fungus from the test tubes, 5 ml of a sterile saline solution (NaCl) were added (0.85% in distilled water), the mixture was shaken gently and the volume required extracted for inoculation of the culture media selected for the study.

### General Activator of Metabolism in Plants (GAMP):

To produce AGMP, samples of approximately 1-2 ml of the culture medium with mycelium were inoculated into 150 ml of six different modified media, described originally by Calam and Nixon (cited by Kumar and Lonsane 1989). Those media contained a common base of ammonium acetate (5 g/litre of culture medium) as source of nitrogen and 1 ml of a Grofol MR (1) solution containing 1 g of the commercial product per litre of water as general source of minerals. The media were prepared, varying the quantity (20 and 50 g/ litre) of the source of carbohydrates: glucose, lactose or saccharose, depending upon the treatment. All the media were adjusted to pH 5.5 with 1 N NaOH and 1 N HCl as required. In duplicate the media were placed in 250 ml Erlenmeyer flasks, sterilized in an autoclave at 15 pounds of pressure and at 120°C for 15 minutes. The inoculum was then incubated, remaining stationary, for 5,7,10, 13, 15, 17, 20, 22 and 25 days at a temperature of 25-50°C. The resulting product of this fermentation is the GENERAL ACTIVATOR OF GROWTH IN PLANTS (GAMP) and the following was carried out:
a) The resulting media were immediately refrigerated at 2°C and subsequently dehydrated at ambient temperature (Table 6).
b) The other media obtained were kept refrigerated at 2°C for carrying out different assays.
c) The resultant GAMP product can be kept indefinitely as a dry product by dehydration or can be preserved in alcohol.

To distinguish between the treatments, an identification key has been given to each treatment, using a number to denote the quantity in grams of the source of carbohydrate, followed by the first letter of the name of the carbohydrate and finally a number to indicate the period of incubation in days. Thus, the key 20G5 means the treatment containing 20 grams of glucose and an incubation period of 5 days, and so on.

### Obtaining the GAMPa :

A product called GAMPa was separated from the GAMP liquid culture media using the following sequence. Once the fermentation process was complete, volumes of 20 ml of the medium were adjusted to pH 3 by slowly adding a solution of 1N HCl. The mixtures were then transferred to separating funnels and 20 ml of ethyl acetate were added. The mixture was stirred and the aqueous organic phase was separated out, the latter being transferred to a separating flask. The aqueous volume recovered was treated three more times with the same volume of ethyl acetate, repeating the separation to recover all of the GAMPa of the original sample. Once combined, the volumes of ethyl acetate were adjusted to a pH of 7 and in a rotary evaporator the solvent was evaporated off until the smallest volume possible was obtained. The final residue (hereinafter called the GAMPa solid extract) was transferred to a beaker and kept at ambient temperature for the subsequent assays.

### Chemical identification

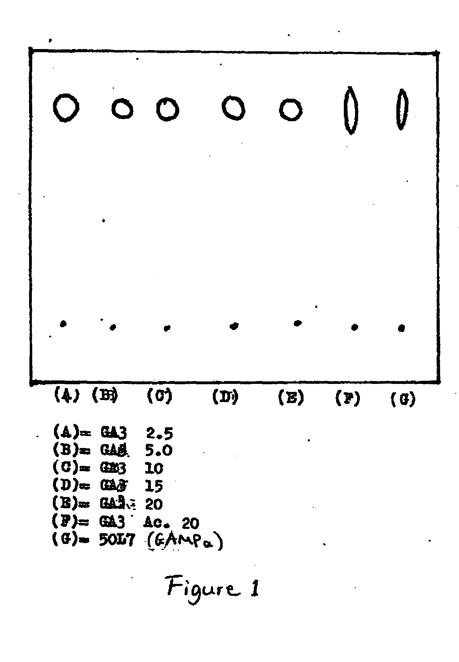

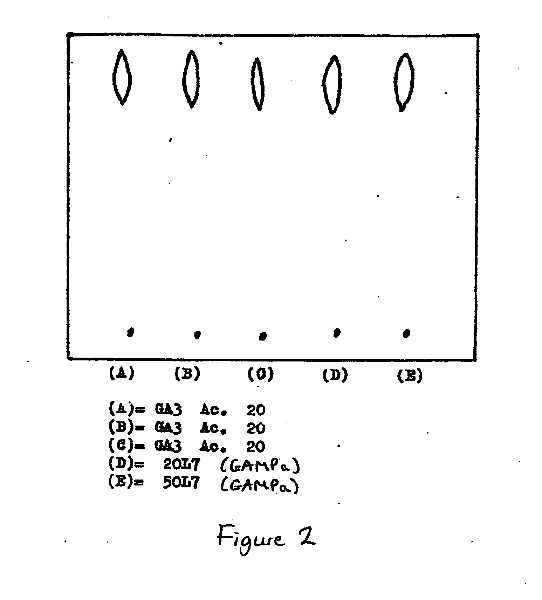

The chemical verification of the presence of GAMPa in the final residue extracted in the manner described in the preceding paragraph was carried out using the technique of separation by thin-layer chromatography and detection by fluorescence described by Kumar and Lonsane (1986).
The solid extract sample that was recovered from the distillation process was resuspended in 4 ml of ethyl acetate. As control, solutions of a known growth regulator, pure GA₃ (Sigma Chemical, Co. cat. G 7645; 1993) were prepared in order to carry out a comparison with another known pure product, containing 1.25, 2.5, 5, 10, 15 and 20 mg/ml in ethanol. Using a capillary tube, a small drop (volume not determined) of the standard suspensions and of the suspensions to be tested was placed on the starting line of a silica plate, in a solvent medium of benzene: acetone: acetic acid (13:6:1). The samples were left to migrate in the solvent to approximately 8 cm from the edge of the plate. Once the solvent had evaporated, the plate was sprinkled with a solution of sulphuric acid (70%) to visualize the resultant spots in a dark room under the incidence of ultraviolet light. (Figures 1 and 2)

### High-resolution liquid chromatography

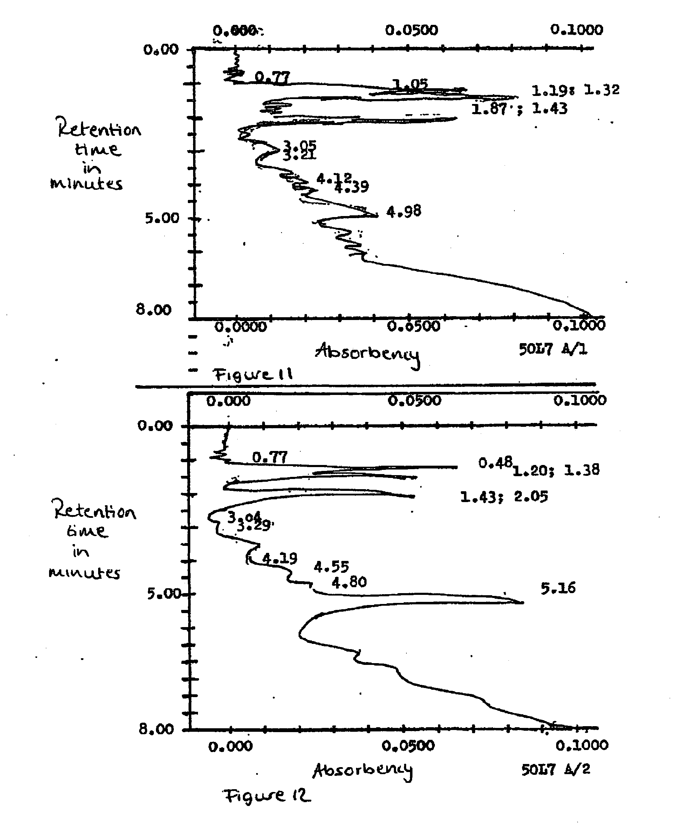

For this analysis -according to data from the laboratory that carried out the study- an ODS column was used with a UV/VIS detector with a diode array of 210 nm and scanning at 200-350 nm. The samples provided at the laboratory were prepared from volumes of 20 ml of the fermentation liquid media corresponding to treatments 50L7 (two samples) and 50L10 (three samples), which were subjected to the extraction process using ethyl acetate already described to obtain GAMPa. This is a comparative assay between GAMPa and GA₃ and was carried out using the technique of adding a standard by adding 20 µl of concentrated GA₃ standard to 200 µl of sample (Figures 11 and 12).

### Quantitative analysis

The quantitative determination of the GAMPa content in the liquid medium resulting from the fermentation process (GAMP) obtained in the form of solid residues (GAMPa) from the extraction with ethyl acetate, with 20 ml samples of the fermentation liquid medium (GAMP), was carried out by spectrophotometric assay (Holbrook 1960).

### Spectrophotometry

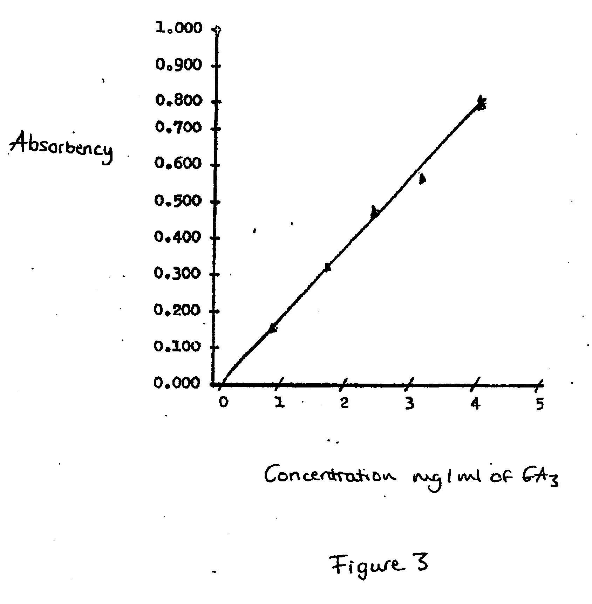

The spectrophotometric analysis was carried out in the following manner. A sample obtained by treatment of the solid extracts (GAMPa) obtained from 20 ml of each fermentation medium (GAMP) was dissolved in 100 ml of absolute ethyl alcohol. A volume of 10 ml of each alcoholic suspension was then diluted with 90 ml of a 30% solution of HCl and left in a beaker of water at ambient temperature for 75 minutes. To draw the regression line of absorbency vs. concentration of GA₃, solutions containing 0.8, 1.6, 2.4, 3.2 and 4 mg of GA₃ in a final solvent volume of 100 ml were prepared from a mother solution of GA₃ in absolute ethyl alcohol as indicated in Table 1. The spectrophotometer was calibrated in zeros with various blanks, using 10 ml of absolute alcohol and 90 ml of 30% HCl for the GA₃ standard and a mixture of 35 ml of 5% HCl and 65 ml of water for the solutions to be studied. The absorbency of the solutions was quantified at 254 nm using a Beckman digital spectrophotometer (Holbrook (1961) (Figures 3, 4, 5, 6).

**Table 3. Dilutions of gibberellic acid (GA₃) prepared from a mother solution containing 0.4 mg/litre of acid in absolute ethyl alcohol, used to draw the regression line of absorbency of GA₃.**

| Mother solution (GA₃) (ml) | Absolute Et-OH ml | 30% HCl (ml) | mg/GAMP in 100 ml |
|---|---|---|---|
| 2 | 8 | 90 | 0.8 |
| 4 | 6 | 90 | 1.6 |
| 6 | 4 | 90 | 2.4 |
| 8 | 2 | 90 | 3.2 |
| 10 | 0 | 90 | 4.2 |
| Blank | 10 | 90 | |

### ASSAY OF THE BIOLOGICAL EFFECT

### A) Bioassays were carried out using the GAMPa extracted with ethyl acetate.

Demonstration of the biological effect of the crude extracts obtained was carried out only using the samples of the treatments (GAMP) 20G7, 20L15 and 50L10 which produced the most GAMP according to the spectrophotometric analysis. For this purpose, techniques stimulating the production of alpha-amylase were used (Fernandez and Johnston 1986) in corn seed and elongation of the hypocotyl in recently germinated lettuce plants (Lactuca sativa) (Robalo and Rojas 1982) and substitution of the light requirements in the germination of lettuce (Robalo and Rojas 1982).

### Production of alpha-amylase

The stimulating effect of GAMPa upon the synthesis of alpha-amylase was carried out according to the following procedure. Starting from a mother solution containing 1 mg/ml of GA₃ in water (before diluting with water, the volume of GA₃ is dissolved in 1 ml of ethanol), control dilutions were prepared containing 0.1, 1, 10 and 100 ppm of GA₃ in an aqueous medium in turn containing 0.7% of Phytagel MR and 0.15% of starch for iodometry. Given that this medium gels very quickly, volumes of 10 ml of the suspension were rapidly poured into Petri dishes to prepare the assay. In the same manner, the solid extracts obtained from 20 ml of the GAMPa fermentation medium were dissolved in 100 ml of absolute ethyl alcohol and the following dilutions were prepared in water: 1:10, 1:100, 1:1000 and 1:10000. A milliliter of each dilution was then mixed with 9 ml of the suspension of Phytagel MR and starch.

### MR agar substitute gelling agent, Sigma Chemical Co. Cat. P8169

To detect and compare the alpha-amylase activity, a certain quantity of corn seeds was soaked in a 50% H₂SO₄ solution for 1 hour, at the end of which the seeds were transferred to a container containing water, where they were left for 24 hours. Once that period had elapsed, the seed embryo was removed and cut longitudinally through the middle, each seed half being placed in a dish with the portion of the endosperm in contact with the medium. After 24 hours' incubation, the seeds were removed and Lugol's solution was added to the medium to detect the presence of clear areas around the seeds, indicating the absence of starch.

**Lengthening of the hypocotyl of lettuce:** Starting from a mother solution containing 1 mg/ml of GA₃ in water (the volume of GA₃ was initially diluted in 1 ml of ethanol) dilutions containing 0.1, 0.15, 0.2 and 1 ppm in distilled water were prepared, which were the GA₃ controls. From the GAMPa, which is in the form of solid extracts dissolved in absolute ethanol as already described, dilutions of 1:1000, 1:5000, 1:7000 and 1:10000 in water were prepared. Five mililitres of each were then poured into 16 Petri dishes, each provided with a few filter papers in the base. Two replicas with distilled water only were used as control. 20 lettuce seeds were placed in each dish, which were left to incubate in darkness for 48 hours, at the end of which the length of the hypocotyls was measured (Table 4A and B).

**Substitution of light requirements during germination.** The procedure for carrying out this assay is identical to the assay on the lengthening of the hypocotyl described above, with the only exception that the measurement of the growth of the hypocotyl was carried out at the end of the germination period in darkness. Solutions containing 1,10 and 100 ppm of GA₃ and dilutions of : 10, 1:100 and 1:1000 of the solid extracts of the assay (GAMPa) dissolved in absolute ethanol as already described were used for this assay.

B) **Bioassays carried out with the GAMP obtained by fermentation.** The demonstration of the biological effect of the crude Growth Regulating Product (GAMP) obtained was carried out with all the samples of the treatments (GAMP) 20G5, 20G7, 20G10, 20G15, 50G5, 50G7, 50G10, 50G15, 20L5, 20L7, 20L10, 20L10, 50L5, 50L7, 50L10, 50L15, 20S5, 20S7, 20S10, 20S15, 50S5, 50S7, 50S10, 50S15, dilutions of 1:10 - 1:10 000 000 000 000 000 being prepared in water to test for the production of alpha-amylase (Fernandez and Johnston 1986) in corn seed, elongation of the hypocotyl of recently germinated lettuce (Lactuca sativa) (Robalo and Rojas 1982) and substitution of the light requirements in the germination of lettuce (Robalo and Rojas 1982). (Figures 5-8)

### Production of alpha-amylase

The stimulating effect of GAMP upon the synthesis of alpha-amylase was carried out according to the following procedure. Starting from a mother solution containing 1 mg/ml of GAMP in water (before dilution with water, the volume of GRP was dissolved in 1 ml of ethanol), control dilutions were prepared containing 0.1, 1, 10 and 100 ppm of GA₃ in an aqueous medium itself containing 0.7% Phytagel MR and 0.15% starch for iodometry. Given that the medium forms a gel very quickly, volumes of 10 ml of the suspension were poured rapidly into Petri dishes to prepare the assay. Solutions of dilutions in water of GAMP of 1:10 - 1: 10 000 000 000 000 000. A milliliter of each dilution was then mixed with 9 ml of the suspension of Phytagel MR and starch.

MR agar substitute gelling agent, Sigma Chemical Co. Cat. P8169

To detect and compare the alpha-amylase activity, a certain quantity of corn seeds was soaked in a 50% H₂SO₄ solution for 1 hour, at the end of which the seeds were transferred to a container of water where they were left for 24 hours. Once that period of time had elapsed, the seed embryos were removed and cut longitudinally through the middle, each seed half being placed in a dish with the portion of the endosperm in contact with the medium. After 24 hours' incubation, the seeds were removed and Lugol's solution was added to the medium to detect the presence of clear areas around the seeds, indicating the absence of starch.

**Lengthening of the hypocotyl of lettuce.** Starting from a mother solution containing 1 mg/ml of GA₃ in water (the volume of GA₃ was initially diluted in 1 ml of ethanol), dilutions were prepared containing 0.1, 0.15, 0.2 and 1 ppm in distilled water, which were the GA₃ controls. Dilutions of the Growth Regulating Product (GAMP) were made. Solutions of dilutions in water of GAMP in a range of 1:10 to 1:10 000 000 000 000 000. Two replicas with distilled water only were used as control and GA₃ was used as positive control. 20 lettuce seeds were placed in each dish, which were left to incubate in darkness for 48 hours, at the end of which the length of the hypocotyls was measured.

**Substitution of light requirements during germination.** The procedure for carrying out this assay is identical to the assay for the lengthening of the hypocotyl described above, with the only exception that the growth of the hypocotyl is measured at the end of the germination period in darkness. Solutions of diliutions in water of GAMP were in the range of from 1:10 to 1:10 000 000 000 000 000.

### RESULTS AND DISCUSSION

The following contains the results of the chemical analyses and assays of the biological activity of the product of submerged fermentation with a strain of *Fusarium moniliforme,* which I have called General Activator of Metabolism in Plants (GAMP) and of a product extracted therefrom GAMPa. In the process of fermentation of GAMP, there were variations, both qualitative and quantitative, such as the source of carbohydrate and the fermentation time, and the time and temperature of incubation.

### Chemical identification

Chromatography. Copies to scale of the chromatograms obtained using samples of GAMPa isolated with ethyl acetate from the solid extracts of treatments 50L7(GAMP) and 20L15 (GAMP) are shown in Figures 1 and 2. Unlike the other treatments, with these two good lyophilisates were obtained which were easy to handle on account of their consistency. It was not considered necessary to reproduce the chromatograms of the other 22 treatments since all yielded the same result. The Rf calculated for GA₃ in the solvent medium used is 0.84. The plates containing GA₃ in Et-OH, GA₃ in acidified Et-OH and solid extract (GAMPa) in ethyl acetate, once developed with H₂SO₄, clearly showed the fluorescent stains corresponding to the presence of GAMP, the shape of which is different from that of GA₃ (control) when visualised under the incidence of ultraviolet light (Cross 1954) (Figure 1 and 2).

### High-resolution liquid chromatography

The chromatogram of the standard solution of GA₃ showed a main peak at 4.96. The direct injection of the sample of Growth Regulating Product (GAMPa) presented a complex composition with peaks around 4.96. This proved that there was no gibberellic acid present in our samples or that the quantity present was not significant (Figures 11 and 12).

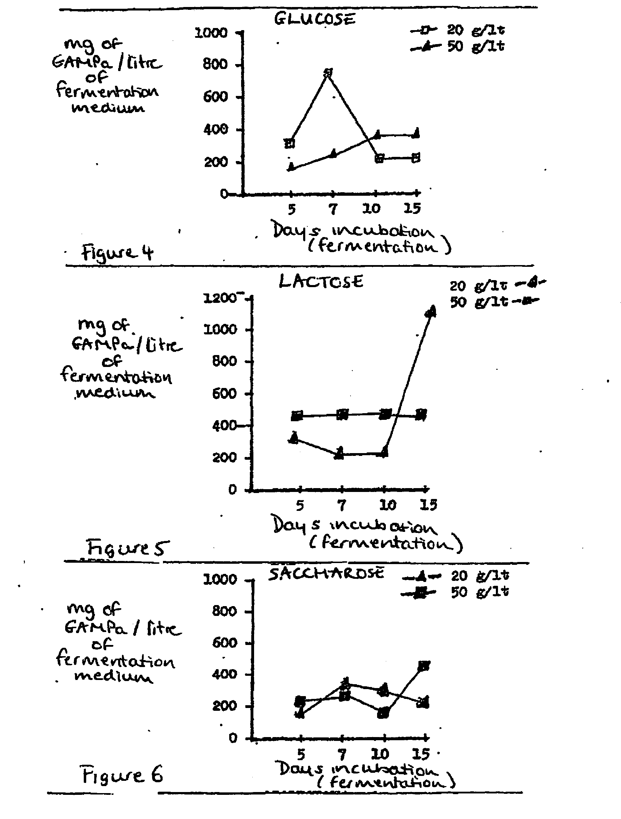

Spectrophotometry. The values for average absorbency obtained with the two samples of solid extract of each treatment and their equivalences in weight of GA₃ (Table 5) were calculated starting from a standard curve roughly adjusted (Figure 3). Thus, Table 5 contains the average yield values for GAMPa adjusted by period of incubation and by concentration and type of source of carbohydrates in the fermentation medium (Gohlwar *et al* 1984). The values from Table 5 were in turn used to plot the tendency of production curves shown in Figures 4, 5 and 6, which makes it possible to appreciate that there is no definitive consistent tendency in the relationship of fermentation time to production of GAMPa when different substrates are compared. The same result is obtained if the comparison is made using the same substrate but varying the content of the sugar in the fermentation medium. In the latter case, no appreciable difference can be detected in the size of the yield of GAMPa in the different fermentation times comparing two concentrations from the same source of carbohydrates.

Table I. Effect of standard GA₃ and of 4 dilutions of the solid extracts (GAMPa) resulting from treatments 20G7 (20 g/l, 7 days' fermentation), 20L15(20 g/I of lactose, 15 days' fermentation) and 50L10 (50 g/l of lactose, 10 days' fermentation) upon the length of hypocotyls of lettuce (A), and estimated equivalents of GA₃ (ppm) corresponding to each dilution, according to the spectrophotometric analysis of the samples of solid extract from each treatment B.
(A)

| Treatment | Length of hypocotyl in milimetres | | | |
|---|---|---|---|---|
| | 1:1000 | 1:5000 | 1:7000 | 1:10 000 |
| | | | | |
| GA₃ | 28 | 27 | 25 | 28 |
| GAMPa 20G7 | 22 | 28 | 27 | 28 |
| GAMPa 20L15 | 26 | 32 | 28 | 30 |
| GAMPa 20L 10 | 27 | 27 | 25 | 20 |
| Control | 12 | | | |
| | | | | |

(B)

| Treatment | Equivalence in ppm between GA₃ and GAMPa according to the corresponding dilution | | | |
|---|---|---|---|---|
| | 1:1000 | 1:5000 | 1:7000 | 1:10 000 |
| | | | | |
| GA₃ | 1 | 0.2 | 0.15 | 0.1 |
| GAMPa 20G7 | 0.15 | 0.03 | 0.02 | 0.015 |
| GAMPa 20L15 | 0.225 | 0.045 | 0.032 | 0.025 |
| GAMPa 50L10 | 0.106 | 0.0212 | 0.0151 | 0.0106 |

Table 4. Average values of absorbency and their equivalents in mg of Growth Regulating Product (GAMPa)/litre, obtained with samples of aliquots of the GAMPa solid extract resulting from the submerged fermentation process from *Fusarium moniliforme* on different substrates and with different incubation times. This is a method for quantifying GA₃ in solid samples compared with GAMPa from the resultant GAMP liquid medium.

| *Treatment (1) GAMPa isolated from GAMP from different sources of sugar and with different incubation times* | Equivalent at 254 nm | Absorbency GA₃ (mg / litre) |
|---|---|---|
| Glucose | | |
| 20G5 | 0.7 | 350 |
| 20G7 | 1.50 | 750 |
| 20G10 | 0.47 | 235 |
| 20G15 | 0.47 | 237 |
| 50G5 | 0.35 | 175 |
| 50G7 | 0.54 | 270 |
| 50G10 | 0.78 | 390 |
| 50G15 | 0.78 | 390 |

| Lactose | | |
|---|---|---|
| 20L5 | 0.70 | 350 |
| 20L7 | 0.50 | 250 |
| 20L10 | 0.59 | 295 |
| 20L15 | 2.25 | 1125 |
| 50L5 | 0.94 | 470 |
| 50L7 | 0.94 | 470 |
| 50L10 | 1.00 | 500 |
| 50L15 | 1.06 | 530 |

| Saccharose | | |
|---|---|---|
| 20S5 | 0.41 | 205 |
| 20S7 | 0.65 | 325 |
| 20S10 | 0.55 | 275 |
| 20S15 | 0.47 | 235 |
| 50S5 | 0.47 | 235 |
| 50S7 | 0.47 | 235 |
| 50S10 | 0.24 | 120 |
| 50S15 | 0.24 | 470 |

| | | |
|---|---|---|
| (1) The first number indicates the quantity (grams/litre) of source of carbohydrate in the culture medium: G = glucose, L = lactose and S = saccharose; the number to the right of the letter indicates the fermentation time in days. | | |

No substantial difference was detected when the comparison was made between different sources of sugar. Given that it was not possible to find any bibliographical references containing specific information relating to the curve for the tendency of production of GAMPa as a function of fermentation time, it is not possible to compare the results of this study to see if the tendencies observed and the yields match a previously observed pattern. In relation to the yield of GAMPa, if the two values that differ from the range within which the rest of the treatments fall are eliminated (corresponding to treatments 20G7 and 20L15), glucose (both 20 and 50 g/I) varies approximately within the range from 175-390 mg/l, that is to say by 215 units; lactose is within a range of 250-530 mg/l, or 280 units; and saccharose varies within a range of 120-470, corresponding to 350 units. The corresponding mean values for the size of variation are 282.5, 390 and 295 for the treatments with glucose, lactose and saccharose, respectively; which makes one think that there are no real differences between treatments.

### Assay of the biological effect

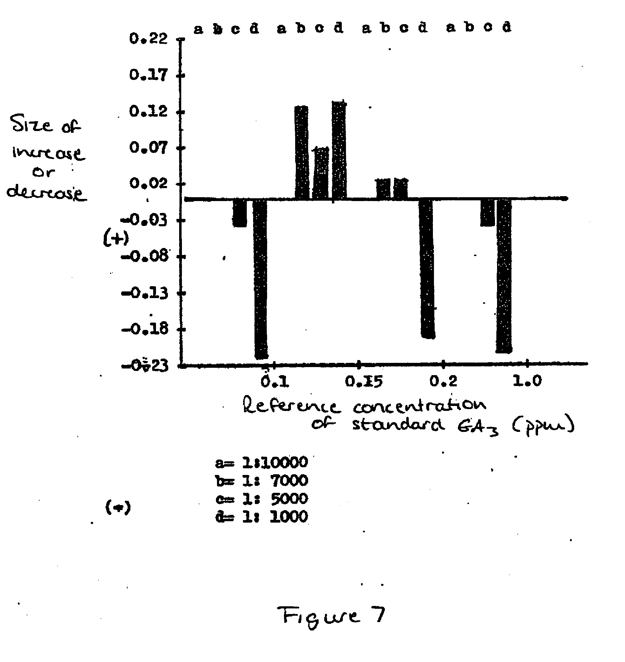

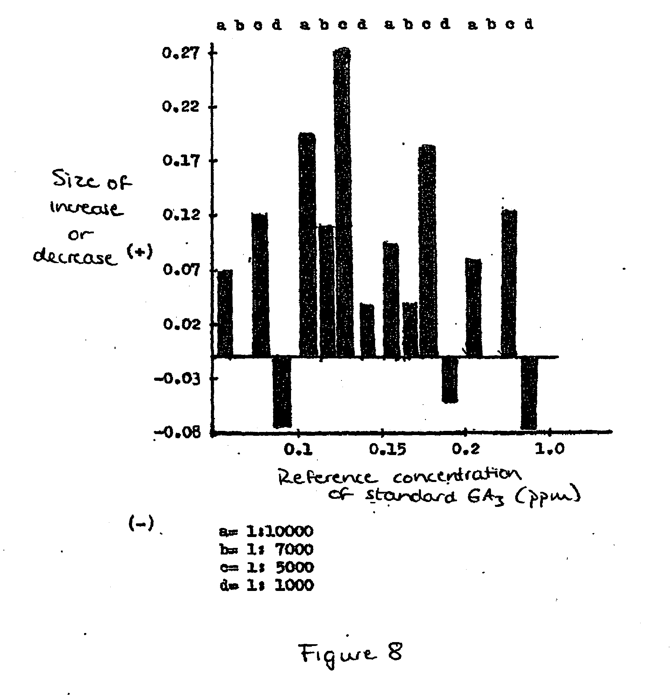

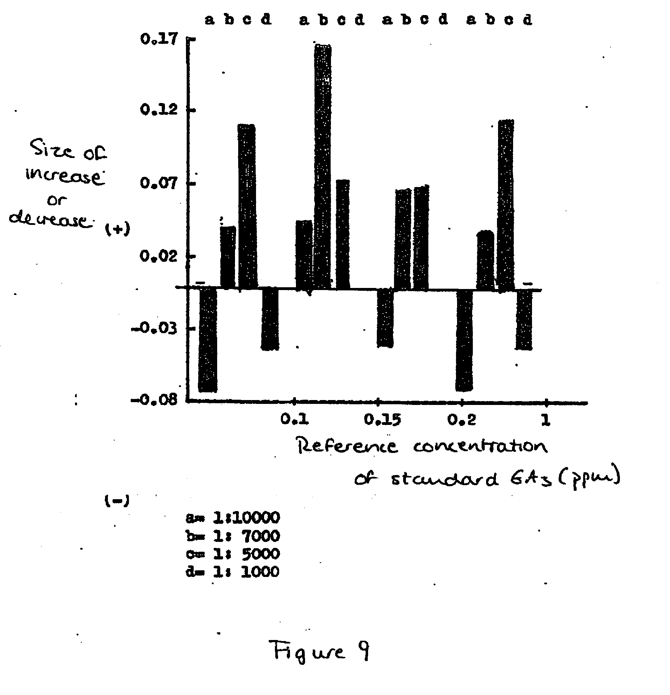

*Induction* of *the elongation* of *the hypocotyl.* Effect of the GAMPa solid extracts corresponding to treatments 20G7, 20L15 and 50L10 called GAMP (the treatments which yielded the best yield according to the technique of Holbrook 1961) on the length of the hypocotyl of lettuce plants (Figures 7-9). For reasons of time, the rest of the treatments were not evaluated. Four volume to volume dilutions of the GAMPa solid extract in ethanol of 1:1000, 1:5000 ,1:7000 and 1:10000 were assayed and their effect was expressed in terms of magnitude (times) the decrease or increase in the average growth of the hypocotyl compared with four concentrations of the standard (pure GA₃) and an absolute control (without GA₃). In Figure 7 which shows in graph form the effect of the dilutions originating from the treatment 20G7 (GAMPa) on the increase or decrease in the hypocotyl, it was observed that the dilutions 1:10000 and 1:5000 have a similar effect; both inducing greater elongation of the hypocotyl compared with the concentrations 0.15 and 2 ppm of GA₃, and equal to the concentrations 0.1 and 1 ppm of the acid. The dilution 1:7000 produces an elongation of the hypocotyl greater than that of the control on its own in the concentration 0.15 ppm of GA₃, a response equal to the control at 0.2 ppm and a response less than the control at 0.1 and 1 ppm. The dilution 1:1000 induced in all cases elongation less than all the concentrations of GAMPa. Equally, Figure 8 shows the effect of the dilutions of GAMPa from the treatment 20L15 (20 g/l of lactose, 15 days' fermentation). The dilutions 1:10000 and 1:5000 have a greater effect than the GA₃ controls, the dilution 1:7000 has an effect greater than the concentrations of 0.15 and 0.2 ppm of GA₃ and an equal effect to the concentrations of 0.1 and 1 ppm of the control. The dilution 1:100 on its own has a greater effect when compared with the concentration of 0.15 ppm of the control. Finally, Figure 9 shows the graph corresponding to (GAMPa) of the treatment 50L10. In this graph it can be observed that the dilution 1:10000 has a greater effect than the concentrations of the control, dilution 1:7000 has an effect comparatively greater than the concentrations 0.1, 0.2 and 1 ppm, and an effect equal to the concentration 0.15 ppm of GA₃. (GAMPa) dilutions of 1:5000 and 1:1000 show similar behaviour, having a greater effect than the concentration of 0.15 ppm, an equal effect to the concentration 0.2 and a lesser effect than concentrations 0.1 and 1 ppm of GA₃. Figure 8 shows the tendency of the response of the lettuce plants to the stimulus of the dilutions of the GAMPa solid extracts of treatments 20L7, 20L15 and 50L10, as well as to the different concentrations of the standard solution of GA₃. Given that the graphs have been plotted with few points, the response curves form a semi-log. This Figure shows that GA₃ has its maximum effect at concentrations of 1 and 0.1 ppm (average length of hypocotyl of 28 mm), and the least effect with the concentration 0.15 ppm (average growth of 27 mm). The maximum effect of GA₃ on the elongation of the hypocotyl was exceeded by dilutions 1:5000 and 1:10000 of GAMPa of treatment 20L15 and dilution 1:10000 of GAMPa of treatment 50L10 and 1:10000 of GAMPa of 50L10. The rest of the treatments (except 1:10000 of treatment 20G7) were below the intensity of the response induced by the best concentration of GA₃.

Stimulation of germination. Table 1 shows the effect of three volume to volume dilutions of the GAMPa solid extracts corresponding to treatments 20L7, 20L15 and 50L10 on the germination of lettuce seeds. These dilutions were prepared from a 1:100 dilution in ET-OH of the solid extract resulting from extraction with ethyl acetate carried out in 20 ml of culture medium. Additionally, dilutions containing 1, 10 and 100 ppm of standard GA₃ in water were used to compare the resulting effect . In the three treatments it is observed that the 1:10 dilution inhibits germination totally. Dilution 1:1000 promotes it almost totally and dilution 1:100 promotes it on a scale approximately midway between the previous two. As for the growth of the hypocotyl, the concentration 1:1000 induces double the growth in the three treatments when compared with dilution 1:100. With regard to the effect of the dilutions of GA₃, the acid induces 100% germination at concentrations of 1 and 10 ppm, whilst at the greater concentration (100 ppm) germination is 95 %. The same occurs with the growth of the hypocotyl.

Table 1^{a}. - Effect of dilutions of the GAMPa solid extract on germination and elongation of the hypocotyl of lettuce plants after 72 hours. Average of 20 repetitions per treatment. The control (water) showed 100% germination and a hypocotyl length of 12 mm.

| Treatment (1) | Average percentage of germination and length (2) of hypocotyl according to dilution: | | | | | |
|---|---|---|---|---|---|---|
| | 1:10 | | 1:100 | | 1:1000 | |
| | % germ. | length | % germ. | Length | % germ. | length |
| 20G7 | 15 ppm | 15 ppm | 1.5 ppm | 1.5 ppm | 0.15 ppm | 0.15 ppm |
| | 0 | - | 35 | 4 | 90 | 8 |
| 20L15 | 22.5 ppm | 22.5 ppm | 2.25 ppm | 2.25 ppm | 0.225 ppm | 0.225 ppm |
| | 0 | - | 35 | 4 | 90 | 8 |
| 50L10 | 10.6 ppm | 10.6 ppm | 1.06 ppm | 1.06 ppm | 0.106 ppm | 0.106 ppm |
| | 0 | - | 40 | 4 | 90 | 7 |
| GA₃ | 100 ppm | | 10 ppm | | 1 ppm | |
| | 95 | 7 | 100 | 13 | 100 | 13 |

| | | | | | | |
|---|---|---|---|---|---|---|
| (1) 20G7 = 20 g/I of glucose, 7 days' fermentation | | | | | | |
| 20L15 = 20 g/I of lactose, 15 days' fermentation | | | | | | |
| 50L10 = 50 g/l of lactose, 10 days' fermentation | | | | | | |
| GA₃ = gibberellic acid | | | | | | |
| (2) Equivalents in ppm of the corresponding dilution of the treatment specified. | | | | | | |

Effect on alpha-amylase activity. Table 6 shows the amylase activity of corn seeds induced by different dilutions of the GAMPa solid extracts of treatments 20G7, 20L15 and 50L10 (GAMP). For comparison with the control without GA₃, four concentrations of standard GA₃ were assayed. The results indicate a similar response between the GA₃ dilutions and those of treatments 20G7 and 50L10(GAMP). The latter indicated maximum alpha-amylase activity at the 1:100 dilution, which corresponds to the effect of the dilution of 10 ppm of GA₃. The treatment 20L15 induced the maximum activity at a 1:10 dilution, which leads one to suppose that in this treatment the amount of GAMP is less than in the other two.

Table 2^{a}.- Shows the effect of the dilutions of the GAMPa solid extracts obtained by the fermentation of different substrates by the activity of *Fusarium moniliforme* upon the amylase activity of corn seeds by the technique of Robalo and Rojas (1982)

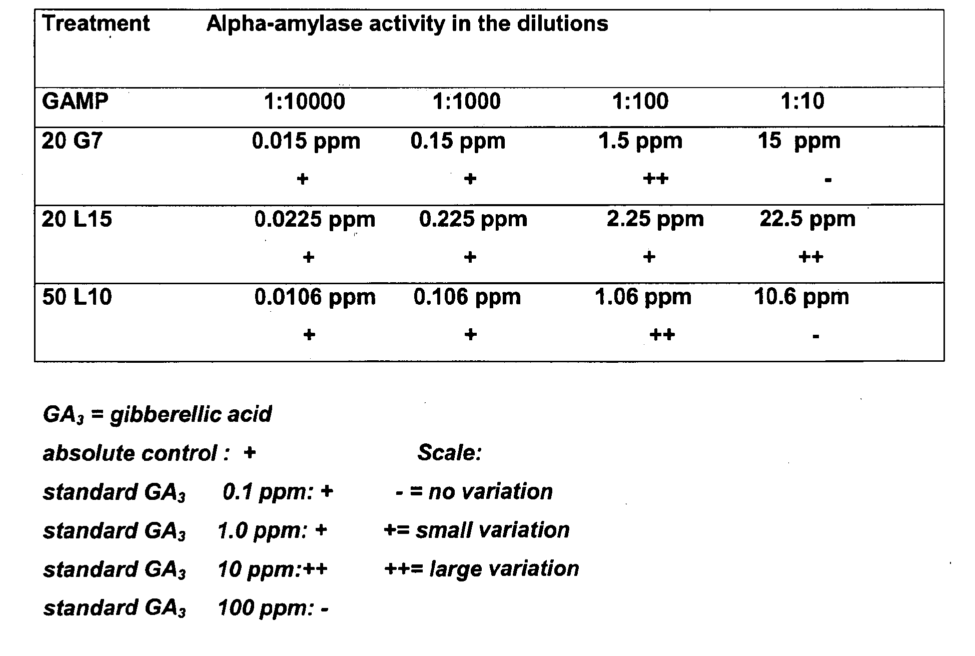

### General Activator of Metabolism in Plants (GAMP)

Evaporation to the environment of the GAMP mother solution (50S7) was carried out. The following results were obtained.

**Table 6: Shows the weight in grams, and consistencies of solid GAMP obtained by evaporation of different samples.**

| **Amount in millilitres of the GAMP mother solution** | **GAMP weight in grams** | **Solid GAMP in 1 litre of GAMP** | **Appearance of the solid** |
|---|---|---|---|
| 800 | 21.4 | 53.5 | **viscous and dark coffee in colour** |
| 800 | 20.5 | 51.25 | **viscous and dark coffee in colour** |
| 800 | 18.2 | 45.5 | **viscous and dark coffee in colour** |
| 800 | 16.17 | 40.42 | **viscous and dark coffee in colour** |
| 800 | 21.6 | 54.0 | **viscous and dark coffee in colour** |
| 800 | 20.0 | 52.5 | **viscous and dark coffee in colour** |
| 800 | 22.6 | 56.5 | **viscous and dark coffee in colour** |
| 800 | 17.6 | 44.5 | **viscous and dark coffee in colour** |

### CONCLUSIONS

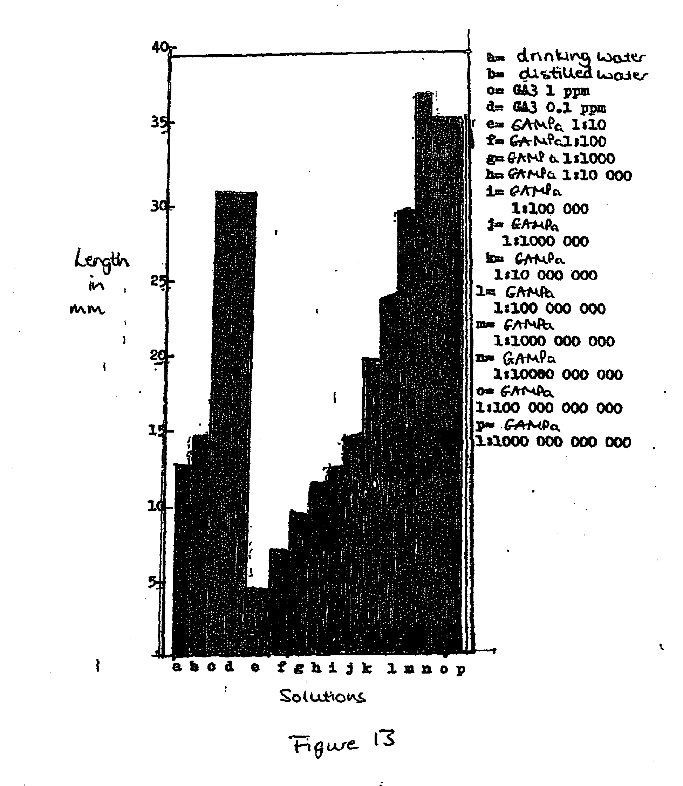

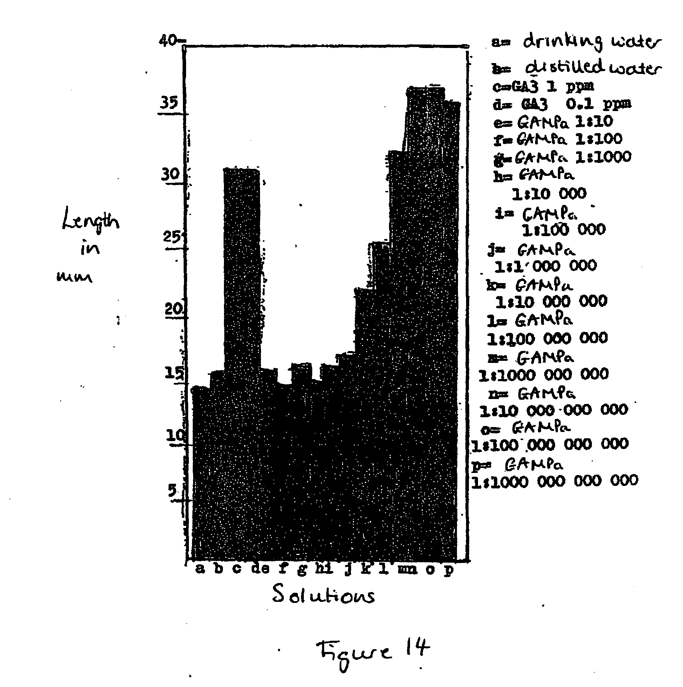

A) GAMPa
   It is concluded that the GAMPa obtained by this process of fermentation used IN DILUTE FORM is more powerful than gibberellic acid (GA₃) in its biological effect on lettuce plants, it being observed that when the product is diluted it causes growth in the plants, having its optimum effect in the lowest dilutions.
   The techniques of thin-layer chromatography and high-resolution liquid chromatography showed the presence of a product other than gibberellic acid GA₃) (Figures 1 and 2).
B) GAMP
   It is also concluded that the General Activator of Metabolism in Plants (GAMP) has its greatest effect when diluted in extremely small quantities such as 1: 10 000 0000, WHICH SUGGESTS THE PRESENCE OF A NEW PRODUCT OR OF AN INHIBITOR OR SUPPRESSOR SUCH AS FUSARIC ACID (PICOLINIC ACID) WHICH IS PRODUCED AT A TEMPERATURE OF 35 C AND IN A QUANTITY 20 TIMES LESS THAN THE GIBBERELLINS, AND THAT IT HAS A BETTER EFFECT THAN ANY KNOWN GROWTH REGULATOR WHEN DILUTED, WHICH IS WHY THIS PRODUCT HAS TO BE USED IN A DIFFERENT AND UNIQUE MANNER. In comparison with other growth regulators such as GA₃ which was also assayed in the field (Tables 7 and 8; Figures 13 and 14).

### BIOASSAYS CARRIED OUT

### PREPARATION OF SOLUTIONS

A) Preparation of GA₃ which is used in the field, diluted to 10%, yielding a concentration of 1 mg/ml.
   1. 100 mg of GA₃ (10% on an inert support) are weighed out.
   2. 10 ml of ethanol are added.
   3. The product is then bottled, labelled and stored in a refrigerator.

A) Process for the preparation of pure GA₃ concentrate. 1 mg/ml.
   1. 10 mg of GA₃ are weighed out.
   2. 10 ml of ethanol are added.
   3. The product is bottled, labelled and stored in a refrigerator.
C) Solutions of GAMPa (originating from 20 ml of GAMP medium and extracted with ethyl acetate), the resultant GAMPa solid extract was dissolved in ethanol in the smallest volume possible (1 ml) and the following dilutions were made, and labelled with the letters Sln and a number:

**Table of solutions of GAMP**

| Number of Solution (SIn) | Corresponding dilution |
|---|---|
| SIn 18 | 1:10 |
| SIn 17 | 1:100 |
| SIn 1 6 | 1:1000 |
| SIn 11 | 1:10 000 |
| SIn 7 | 1: 100 000 |
| SIn 1 | 1:1 000 000 |
| SIn 2 | 1:10 000 1000 |
| SIn 3 | 1:100 000 000 |
| SIn 4 | 1:1000 000 000 |
| Sln 8 | 1: 2000 000 000 |
| Sln 14 | 1: 3000 000 000 |
| Sln 5 | 1:10 000 000 000 |
| Sln 9 | 1:4000 000 000 |
| Sln 10 | 1:5000 000 000 |
| Sln 6 | 1:6666 000 000 |
| SIn 12 | 1:7000 000 000 |
| Sln 13 | 1: 8000 000 000 |
| Sln 15 | 1:9000 000 000 |
| Sln 19 | 1:100 000 000 000 |
| Sln 20 | 1:1000 000 000 000 |
| Sln 21 | 1:10 000 000 000 000 |
| Sln 22 | 1:100 000 000 000 000 |
| Sln 23 | 1:1000 000 000 000 000 |
| SIn 24 | 1:10 000 000 000 000 000 |

### BIOASSAY WITH LETTUCE SEEDS

### Procedure for the bioassay

1.- 2 solutions of commercial GA₃ are prepared, one diluted to 10 %, which is labelled as GA₃, and the other not being diluted and which is labelled as pure GA₃; these are the positive controls.
2.- Dilutions are prepared in a test tube according to the required solution of GAMPa.
3.- Filter paper is placed in position and 20 lettuce seeds are scattered onto it.
4.- Water, the GA₃ positive controls and the GAMP or GAMPa solutions as appropriate are then added to the Petri dishes.
5.- Incubation is carried out at 30 °C for 5 days
6.-The readings are taken and the averages obtained.

### Results Tables Averages and Figures

### GAMPa- BIOASSAY OF THE HYPOCOTYL

**Table 7.- Shows the effect of solutions of GAMP (diluted and concentrated), observing inhibition of growth with the concentrated solutions and stimulation with the dilute solutions; carried out with respective controls.**

| Solution | Average growth of hypocotyl of lettuce in mm |
|---|---|
| Distilled H₂O | 13 |
| H₂O (drinking water) | 15 |
| GA₃ 0.1 ppm | 31 |
| GA₃ 1 ppm | 31 |
| GAMPa 1:10 | 5 |
| GAMPa 1:100 | 7.5 |
| GAMPa 1:1000 | 10 |
| GAMPa 1:10 000 | 12 |
| GAMPa 1:100 000 | 13 |
| GAMPa 1:1000 000 | 15 |
| GAMPa 1:10 000 000 | 20 |
| GAMPa 1:100 000 000 | 24 |
| GAMPa 1:1000 000 000 | 30 |
| GAMPa 1:10 000 000 000 | 37.5 |
| GAMPa 1:100 000 000 000 | 36 |
| GAMPa 1:1000 000 000 000 | 36 |

### GAMP- BIOASSAY OF THE HYPOCOTYL

### Table of solutions and averages of the GAMP

**Table 8. Shows the effect of GAMP solutions (diluted and concentrated), observing no inhibition of growth with the concentrated solutions; carried out using respective controls.**

| Solution | Average growth of lettuce hypocotyl in mm |
|---|---|
| Distilled H₂O | 13 |
| H₂O (drinking water) | 14 |
| GA₃ 0.1 ppm | 30 |
| GA₃ 1 ppm | 30 |
| GAMP 1:10 | 14 |
| GAMP 1:100 | 13 |
| GAMP 1:1000 | 15 |
| GAMP 1:10 000 | 14 |
| GAMP 1:100 000 | 15 |
| GAMP 1:1000 000 | 16 |
| GAMP 1:10 000 000 | 21 |
| GAMP 1:100 000 000 | 25 |
| GAMP 1:1000 000 000 | 32 |
| GAMP 1:10 000 000 000 | 37 |
| GAMP 1:100 000 000 000 | 37 |
| GAMP 1:1000 000 000 000 | 36 |

### Bioassay in the field

For preparations in the field, calculations were made according to the following Table 3 of solutions:

| Name or number of the Product | Concentration |
|---|---|
| GAMP | Pure product (liquid) |
| GAMP mother solution (starting from this solution + alcohol for the following solutions | GAMP + alcohol (1:1) |
| SIn 18 | 1:10 |
| SIn 17 | 1:100 |
| SIn 16 | 1:1000 |
| SIn 11 | 1:10 000 |
| SIn 7 | 1:100 000 |
| SIn 1 | 1:1000 000 |
| SIn 2 | 1:10 000 000 |
| SIn 3 | 1:100 000 000 |
| SIn 4 | 1:1000 000 000 |
| SIn 8 | 1:10 000 000 000 |
| SIn 14 | 1:100 000 000 000 |
| SIn 5 | 1: 1000 000 000 000 |
| SIn 9 | 1: 4000 000 000 000 |
| SIn 10 | 1: 5000 000 000 000 |
| SIn 6 | 1: 6666 000 000 000 |
| SIn 12 | 1: 7000 000 000 000 |
| SIn 13 | 1: 8000 000 000 000 |
| SIn 15 | 1: 9000 000 000 000 |
| SIn 19 | 1:100 000 000 000 |
| SIn 20 | 1:1000 000 000 000 |
| SIn 21 | 1: 10 000 000 000 000 |
| SIn 22 | 1:100 000 000 000 000 |
| SIn 23 | 1:1000 000 000 000 000 |
| SIn 24 | 1:10 000 000 000 000 000 |

**Table 4 To prepare different quantities of solution 4 (1:1000 000 000)**

| Volume to be prepared | Quantity of solution 4 required |
|---|---|
| 1300 m³ (1 hectare) | Prepare a 1:10 solution of GAMP mother solution (100 ml of GAMP mother solution + 900 ml of alcohol) for 1 litre/ from this solution take 1.3 ml and add to the quantity of water required to irrigate 1 hectare or 1300 m³ of water. |
| 10 m³ | Prepare a 1:100 solution of GAMP mother solution (10 ml of GAMP mother solution + 990 ml of alcohol) for 1 litre/ from this solution take 1 ml and add to 10 m³ of water. |
| 100 litres | Prepare a 1:10 000 solution of GAMP mother solution (0.0001 ml of GAMP mother solution + 999.9 ml of alcohol) for 1 litre/ from this solution take 1 ml and add to 100 litres of water. |
| 30 litres | Prepare a 1:100 000 solution of GAMP mother solution (0.00001 ml of GAMP mother solution + 999.99 ml of alcohol) for 1 litre/ from this solution take 3 ml and add to 30 litres of water. |
| 10 litres | Prepare a 1:100 000 solution of GAMP mother solution (0.00001 ml of GAMP mother solution + 999.99 ml of alcohol) for 1 litre/ from this solution take 1 ml and add to 30 litres of water. |
| 3 litres | Prepare a 1:100 000 solution of GAMP mother solution (0.00001 ml of GAMP mother solution + 999.99 ml of alcohol) for 1 litre/ |
| | from this solution take 3 ml and add to 30 litres of water. |
| 1 litre | Prepare a 1:100 000 solution of GAMP mother solution (0.00001 ml of GAMP mother solution + 999.99 ml of alcohol) for 1 litre/ from this solution take 0.1 ml and add to 30 litres of water. |
| 100 ml | Prepare a 1:1 000 000 solution of GAMP mother solution (0.000001 ml of GAMP mother solution + 999.999 ml of alcohol) for 1 litre/ from this solution take 1 ml and add to 100 ml of water. |
| 10 ml | Prepare a 1:10 000 000 solution of GAMP mother solution (0.0000001 ml of GAMP mother solution + 999.9999 ml of alcohol) for 1 litre/from this solution take 1 ml and add to 10 ml of water. |

### Table 5 to prepare different quantities

### Solution 5 (1:10 000 000 000)

| Volume to be prepared | Quantity of solution 4 required |
|---|---|
| 1300 m³ (1 hectare) | Prepare a 1:100 solution of GAMP mother solution (10 ml of GAMP mother solution + 990 ml of alcohol) for 1 litre/ from this solution take 1.3 ml and add to the quantity of water required to irrigate 1 hectare, or 1300 m³ of water |
| 10 m³ | Prepare a 1:1000 solution of GAMP mother solution (1 ml of GAMP mother solution+ 999 ml of alcohol) for 1 litre/ from this solution take 1 ml and add to 10 m³ of water |
| 100 litres | Prepare a 1:100 000 solution of GAMP mother solution (0.00001 ml of GAMP mother solution + 999.99 ml of alcohol) for 1 litre/ from this solution take 1 ml and add to 100 litres of water. |
| 30 litres | Prepare a 1:1000 000 solution of GAMP mother solution (0.000001 ml of GAMP mother solution + 999.999 ml of alcohol) for 1 litre/ from this solution take 3 ml and add to 30 litres of water. |
| 10 litres | Prepare a 1:1000 000 solution of GAMP mother solution (0.00000 1 ml of GAMP mother solution + 999.99 ml of alcohol) for 1 litre/ from this solution take 1 ml and add to 30 litres of water. |
| 3 litres | Prepare a 1:10 000 000 solution of GAMP mother solution (0.0000001 ml of GAMP mother solution + 999.9999 ml of alcohol) for 1 litre/ from this solution take 3 ml and add to 30 litres of water. |
| 1 litre | Prepare a 1:10 000 000 solution of GAMP mother solution (0.0000001 ml of GAMP mother solution + 999.9999 ml of alcohol) for 1 litre/ from this solution take 1 ml and add to 30 litres of water. |
| 100 ml | Prepare a 1:100 000 000 solution of GAMP mother solution (0.00000001 ml of GAMP mother solution + 999.99999 ml of alcohol) for 1 litre/ from this solution take 1 ml and Add to 100 ml of water. |
| 10 ml | Prepare a 1:1000 000 000 solution of GAMP mother solution (0.000000001 ml of GAMP mother solution + 999.999999 ml of alcohol) for 1 litre/ from this solution take 1 ml and add to 10 ml of water. |

These were the solutions tested in the field in the quantities indicated. Small volumes were prepared using dilutions in order to be able to take the quantity indicated to prepare the corresponding solution.

To ensure that the liquid product has a good appearance 0.1 g/litre of alcohol, which may be ethanol or methanol, of an emerald green colourant used in food products was added. All the solutions can be placed on an inert support such as cotton, paper or fabric, etc., depending on the corresponding solution, and by evaporation the active ingredient is left in the support and for use the cotton is placed in a specific quantity of water and stirred and that water is added together with the support to the final water with which the crop is to be irrigated. The support can be given different colourations depending on the crop.

A study was also carried out on the storage durability of GAMP prepared for use in the field, tests first being carried out in the laboratory on lettuce hypocotyls, which showed an activity of 3 months when prepared in water. The solutions evaluated were solution 4 (1:1 000;000 000) and solution 5 (1:10000 000 000). The experiment was carried out weekly to evaluate each of the solutions and averages were obtained for each month. The result for the fourth month shows only the evaluation of the first week.

**Table 9. Shows the storage durability of GAMP compared with drinking water and distilled water and with GA₃ at 0.1 ppm and 1.0 ppm.**

| Solution | Average growth of the hypocotyl in the first month evaluated each week (mm) | Average growth of the hypocotyl in the second month evaluated each week (mm) | Average growth of the hypocotyl in the third month evaluated each week (mm) | Average growth of the hypocotyl in the fourth month evaluated each week (mm) |
|---|---|---|---|---|
| H₂O distilled | 14 | 15 | 12 | 13 |
| H₂O (drinking water) | 14 | 13 | 14 | 15 |
| GA₃ 0.1 ppm | 29* | 15 | 15 | 14 |
| GA₃ 1.0 ppm | 32* | 15 | 13 | 14 |
| Solution 4 | 33 | 30 | 24 | 14 |
| Solution 5 | 38 | 33 | 28 | 15 |

| | | | | |
|---|---|---|---|---|
| * GA₃ lost its potency the day after its preparation, in ambient conditions. | | | | |

### Application of the above solutions in the field

### RESULTS:

General observations and/or percentages (%). The increase in the corresponding harvest may vary depending on the crop conditions given that in crops with adequate soil and environmental conditions an increase in quality and quantity of the harvest is observed but is more notable in crops that suffer under conditions such as soil untreated with agrochemicals, fertilizers or soil improvers, insufficient water or other adverse conditions, such as flooding, frost, etc., which is why the percentage increase of a harvest can vary from 20-100%.
**A) Citrus fruits**
   - Different tests in this crop showed that solutions 4 and 5 stimulate the following effects in the plants:
   a) an increase in flowering, better fruit set and thus an increase in the productivity of the harvest, in some cases an increase in the size of the fruit.
   b) better quality fruit, better flavour.
   c) compared with the controls, in drought, in similar conditions (without irrigation), resistance to drought was observed. It was possible to pick fruit despite those conditions, the fruit being a little small but very sweet and usable for making juices.
   d) it was observed that when applied to small citrus fruits (size and age) it produces flowering and setting of fruit, and the fruit ripens. In winter it has been observed that leafless trees are full of flowers. Frost resistance has also been observed.
**B) Corn**
   Greater growth of the plant and greater number of cobs (up to 5 per plant) observed in this crop. It also improves the quality of the product in that it increases the quantity of proteins therein, larger grains.
   Stimulates an increase in the harvest with better flavour.
**C) Potato**
   Produces an increase in productivity and uniformity of the potato, also better quantity of good quality potatoes is obtained.
**D) Fig trees**
   In this crop an increase in productivity has been observed throughout the year, with fruit of good flavour, more compact and less perishable. Cuttings were also taken (small portion of the plants were transplanted out of season and the plants took root). More trees obtained.
**F) Pumpkins**
   Increases quantity, quality and resistance to drought conditions.
**G) Tomato**
   Increases the quantity and quality. It was also observed that in flooded conditions the crop survived, it being possible to use the fruit for puree; the fruit from the controls was not recoverable (total harvest lost).
**H) Banana**
   Increases quantity and quality.
**I) Flowers**
   Causes better quality, bigger size and quantity. Also observed that flowers survive in winter as well (seasonal plants) compared with the same plants untreated.
**J) Prickly pears**
   Increases the quantity of prickly pears.
**K) Pear trees.**
   Increases notably the number of flowers and fruit set in such a manner that the fruit grows in clusters and improves the flavour.
**L) Peach trees**
   Increases the number and size and flavour.
**M) Grasses**
   Causes spectacular increases, strong roots observed, and it appears greener and with more grass; can be used to feed livestock or for decorative purposes.
**N) Onions**
   Increases the size, quantity and better flavour.
**O) Apples**
   Enables the production of apples in unfavourable locations at high temperatures of more than 40 °C, the apples produced have excellent flavour and are produced on young trees. It is important to mention that apples require the stimulus of cold weather or a chemical stimulus in order to flower and produce fruit.

### MORE STUDIES TO BE CARRIED OUT

It is necessary to carry out more studies in the field to evaluate all the stress factors which plants encounter, such as:
***a) salinity*** It is feasible that it increases resistance to drought by the development of a deeper and stronger root.
***b) acidity*** (due to acid rain caused by pollution).
***c) frost resistance*** The product has shown that in sensitive plants, there is resistance to this environmental factor. Greater resistance to this factor as a result of stronger development of the plant, given that treated plants have survived the cold, heat and excessive drought.

### A) General Activator of Metabolism in Animals (GAMA) and General Activator of Metabolism in Humans (GAMH)

Also causes desirable or positive effects in animals and human in low dilutions, no dilution being toxic.

### Use of the product in animals:

The product used is solution 4 (1:1,000, 000 000), diluted in liquor of various flavors, in tequila, in ethyl alcohol, in wines, and is administered in 4 or 5 drops to the tongue per day; it should be mentioned that if all of the product is taken at once, it does not cause any harm.
Studies were first carried out on cats, starting with solution 4 (1:1 000 000 000) up to a dilution of 1:1000 (solution 16) orally, it being observed that the cats increased in size and weight, were always very healthy, and a change in behaviour was also observed in that they were affectionate; also an increase in sexual drive and quantity of sexual relations was also observed.
A study was carried out on chickens (50 individuals), they only ate during the day (not at night); an increase in weight of 30% was observed and they had great flavour.
It has also been observed in domestic chickens that were given the solution when young that some grew to be very tall (long legs) and others did not grow so much but their bodies were large. Different behaviour also observed, they were always together and they like being with the people who rear them.
In dogs, aggressivity decreases, they are more affectionate, they grow more and are very healthy.
Solutions were tested ranging from the concentrated GRP (Growth Regulating Product) to solution 4 (1: 1000;000 000) on mice, and no toxic effect was observed.
Great sexual activity was observed, resulting in a large number of litters of very good size.
On the basis of these observations, it was concluded that the product was not toxic and that it has other properties worthy of being exploited

Experiment 1. A study was carried out on 60 mice of both sexes, divided into groups of 20 with the same dietary and temperature conditions. Drinking water was given to the control group, group I was given a 1:100 solution of the GAMA (General Activator of Metabolism in Animals) and group II was given a 1:1000,000 000 solution of the GAMA. The animals were weighed before starting the experiment. They were weighed twice a week and their behaviour was observed
This resulted in an increase in weight and much activity, especially sexual activity.

**Table 10. Shows the results of the increase in weight in mice treated with solution 4 (1:1 000 000 000)**

| GROUP (20 mice per group) | AVERAGE WEIGHT | % increase in weight |
|---|---|---|
| control | 28.25 g | ----- |
| Group I (1:100 GAMA) | 33.06 g | 17% |
| Group II (1:1 000 000 000) | 36.26 g | 28% |

An increase in weight and much sexual activity was observed.

**Experiment 2.** A study was carried out on 40 male chickens, divided into groups of 10, with the same dietary and temperature conditions. Group I, the control, was given drinking water, group II was given water with GAMA diluted to 1:1 000 000 000 and 1 ml therof was injected once a week, group III was injected once a week with 1 ml of a GAMA solution diluted to 1:1 000 000 000 and given drinking water, and group IV was given a GAMA solution diluted to 1 000 000 000. The experiment was ended after 8 weeks when the animals were weighed and sacrificed.

**Table 11. Shows an increase in weight in chickens that were given and/or were Injected with solution 4 (1:1 000 000 000)**

| GROUP | Average initial weight of the chickens | Final weight at the end of 8 weeks | % increase compared with the control |
|---|---|---|---|
| Control Group I | 48.9 g | 1.667 g | ------- |
| Group II (injected and given to drink) | 49.3 g | 2.199 g | 31.9% |
| Group III (injected) | 48.0 g | 2.170 g | 30.0% |
| | | | |
| Group IV (given to drink) | 50.0 g | 2.011 g | 20.0% |

### Humans: General Activator of Metabolism in Humans (GAMH)

On that basis, I began to use the product on myself, in solution 4 (1:1 000 000 000). I observed that I had more energy. After a time, my relatives wanted to use it, with the same results; the young men developed a better body, as if they had been exercising, which is why they called the product "fortachon" (tough guy).
Various people used the product and they all reported that they had a lot of energy and did not tire easily.
A few acquaintances who had impotence problems used it and they reported a return to normality.
In normal people, it causes greater sexual activity in both men and women.
A study of 16 people was carried out, evaluating fatigue, tiredness, sexual activity and behaviour. It was evaluated as follows:

Table 12: An evaluation of different parameters was carried out to measure vitality in humans
+ = good
+ + = very good
+ + + = excellent

| Number of individual | Fatigue | Tiredness | Sexual activity | Behaviour |
|---|---|---|---|---|
| 1 | + | + | ++ | + |
| 2 | ++ | ++ | +++ | + |
| 3 | ++ | + | ++ | ++ |
| 4 | + | +++ | + | ++ |
| 5 | +++ | ++ | +++ | + |
| 6 | + | + | +++ | + |
| 7 | + | + | +++ | + |
| 8 | ++ | +++ | + | + |
| 9 | + | +++ | +++ | +++ |
| 10 | +++ | ++ | ++ | ++ |
| 11 | ++ | + | +++ | + |
| 12 | + | ++ | +++ | + |
| 13 | ++ | + | ++ | + |
| 14 | +++ | + | ++ | + |
| 15 | + | ++ | +++ | + |
| 16 | ++ | ++ | +++ | + |

In general, people reported that they felt very well, with no tiredness, and reported much sexual activity; they also reported that they were happier with their respective partners.

### BIBLIOGRAPHY:

Agrios, G.N. 1989. Fitopatologia. ED. UMUSA. Mexico pp. 80-81.

Alexopoulos, C.J. and C.W.Mims. 1979 Introductory Mycology. Ed. John Wiley & Sons. USA pp. 534-569.

Bird, H.L. and Ch. T. Pugh 1957. A paper chromatographic separations of gibberellic acid and giberellin. Al. Plant Physiology: 33: 45-46

Borrow A., E.G. Jefferys, R.H. J. Kessell, E.C. Lloyd, P.B. Lloyd and I.S. Nixon. 1961. Metabolism of ***Gibberella fujikuroi*** in stirred culture. Canad. J. Microbiol. 7: 227-276.

Bottini, R., G. de Bottini, M. Koshioka, R.P. Pharis and B.G. Coombe et al. 1985: Identification of gibberellins A1, A5, A29 and A32 from immature seeds of apricot (Prunus armeniaca L.) Plant Physiol. 78: 417-419

Cross, B. E. 1954. Gibberellic acid. Part I. J. Chem. Soc. pp. 4670-4676.

Fernandez G.and M. Johnston 1986. Fisiologia Vegetal Experimental. I.I.C.A., San Jose, Costa Rica. p. 239

Fraga, G. 1979. Las giberelinas. Aportaciones al estudio de su ruta biosintica. Fundacion Juan March. Serie Univ. 79. Madrid Espana pp. 5-41

Gohlwar, C.S.. R.P. Sethi, S.S. Marwaha and V.K. Seghal 1984. Gibberellic acid biosynthesis from whey and simulation of cultural parameters. Enzyme Microb. Technol. 6:312-316

Jefferys, E.G. 1970. The gibberellin fermentation. Adv. in Appl. Microb. 13:283-314.

Holbrook, A. A., W. J. W. Edge and F. Bailey. 1961. Spectrophotometric method for determination of gibberellic acid. Adv. Chem. Ser.; 28: 159-167.

Kumar, P.K.R. and B.K. Lonsane. 1986. Spectrofluorodensitometric estimation in thin-layer chromatography of gibberellic acid produced solid-state fermentation. Jour. Chromat. 369:222-226.

Kumar, P.K.R. and B.K. Lonsane. 1987. Extraction of gibberellic acid from dry mouldy bran produced under solid state fermentation. Process Biochem. 22:139-143

Kumar, P.K.R. and B.K. Lonsane 1989: Microbial production of gibberellins state of art. Ferment. Tech. Bioeng. Discipline Cent. Food Technol. Res. Inst., India. 34:29-139

MacMillan, J. and P.J. Suter. 1963. Thin layer chromatography of gibberellins. Nature. 197:790.

MacMillan, J. and N. Takahashi. 1968. Proposed procedure for the allocation of trivial names to the gibberellins. Nature. 217:170-1.

Mitchell J. and G.A. Livingstone. 1973. Metodos para el estudio de hormonas y reguladores del crecimiento. Ed. Trillas, Mexico p. 39

Nakamuro, T. and M. Tsuji. 1974. Gibberellic acid. Chem. Abstr. 81:5

Nakanishi, K., T. Goto, S. Ito, S. Natori and S. Nozoe. 1983. Natural products chemistry. Gibberellins. Vol. 3 Univ. Sci. Books. Mill Valley California, 265-266.

Robalo M.M. and G.M. Rojas. 1982. Fisiologia Vegetal Experimental. ED. UMUSA.Mexico; p. 202

Rojas, G.M. and R.H. Ramirez. 1987. Control hormonal del desarrollo de plantas. Ed. UMUSA; pp. 30-31.

Reeve, D.R. and A. Crozier. 1976. Purification of plant hormone extracts by gel permeation chromatography. Phytochem. 15:791-793:

Sestry, K.S.M, P. Spingh. M.V.V. Srinivasa Rao and C.V.S. Subrahmanyam. 1988. Possibility of utilising industrial residues in gibberellic acid fermentation. Indian Jour. Of Exp. Biol. 26:851-853.

Stanier, Doudoroff and Adelberg. 1970. The Microbial World 3a. Edic. Prentice Hall, USA; p.10.

## Claims

1. A composition obtained by the fermentation of a culture medium with suitable microorganisms, **characterised in that** when diluted it acts as:
a) In plants it causes an inhibitory effect when extracted with ethyl acetate but in direct form (not extracted) it causes no inhibitory effect. Both the solution extracted with ethyl acetate and the solution used directly cause a stimulating effect when highly diluted.
b) When highly diluted, in animals it causes an effect on behaviour and an increase in weight.
c) When highly diluted, in humans it causes an increase in vitality.

2. A composition according to claim 1, **characterised in that** the microorganism used is preferably: *Gibberella fujikori; Fusarium moniliforme; Gibberella zeae; Gibberella sp.; Azetobacter chroococcum; Agrobacterium tumefaciens; Bacillus polymyxa; Torula puclherima* and combinations thereof.

3. A composition according to claim 1, **characterised in that** the dilution is in the range from 1: 1 00 000 000 to 1:10 000 000 000 000 000, and in dilutions 1:1 000 000 000 and 1: 1 0 000 000 000 good effects have been verified.

4. A process for the preparation of the composition according to claims 1 to 3, **characterised by** a culture medium which comprises a source of nitrogen, such as ammonium acetate, a source of carbohydrates, a source of minerals, a pH of 4-7; inoculated with microorganisms, preferably *Gibberella fujikori; Fusarium moniliforme; Gibberella zeae; Fusarium sp.; Azetobacter chroococcum; Agrobacterium tumefaciens; Bacillus polymyxa; Torula pulcherima* and combinations thereof. And a temperature of 30-40°C.

5. The use of a composition according to claims 1 to 3, as:
a) general promoter of growth in plants
b) produces an increase in weight and a positive change in behaviour in animals
c) stimulates vitality in humans and animals.

6. A process that comprises a composition according to claims 1 to 3, **characterised in that** the composition is applied to an inert support and is left to dry.

7. A product according to claim 6, **characterised in that** the support can be: cotton, paper, sponge, synthetic or natural fibres, fabrics, plastic, glass, powders such as talcum, starch.

8. A product according to claims 1 to 3 having high durability and stability of up to 3 months when prepared in water for use on a crop.

9. The use of a composition according to claim 1 as an antioxidant agent in foods and drinks.

10. The use of a composition according to claim 9 wherein the composition is in a dilution of 1:1000 - 1:10 000.
